# EUROPEAN PATENT APPLICATION

(11) **EP 1 918 710 A1**
(43) Date of publication of application: **07.05.2008**
(21) Application number: 07254310.1
(22) Date of filing: 30.10.2007
(51) Int. Cl.: G01N 33/50

(54) **Characterizing prostate cancer**

(30) Priority: 31.10.2006 US 855423 P
(71) Applicant: Veridex, LLC, Warren, NJ 07059 (US)
(72) Inventor: Wang, Haiying, Bridgewater, NJ 08807 (US); Varde, Shobha, Jacksonville, FI 32258 (US); Chowdary, Dondapati, Princeton Junction, NJ 08876 (US); Mehrotra, Jyoti, Bridgewater, NJ 08807 (US); Vener, Tatiana, Sterling, NJ 07908 (US); Mazumder, Abhijit, Basking Ridge, NJ 07920 (US)
(74) Representative: Mercer, Christopher Paul

(57) **Abstract**

Methods and kits for predicting the course or aggressiveness of prostate cancer include detecting the methylation status of various genes.

SEQUENCE LISTING

<110>  VENER, Tatiana
       MEHROTRA, Jyoti
       VARDE, Shobha
       MAZUMDER, Abhijit
       Wang, Haiying
       Chowdary, Dondapati

<120>  CHARACTERIZING PROSTATE CANCER

<130>  VDX5039USPSP

<140>  99/999999
<141>  2006-10-31

<160>  69

<170>  PatentIn version 3.3

<210>  1
<211>  46
<212>  DNA
<213>  Artificial

<220>
<223>  GSTP1 Scorpion


<220>
<221>  modified_base
<222>  (23)..(23)
<223>  -BHQ-HEG-

<220>
<221>  modified_base
<222>  (23)..(23)
<223>  -BHQ (Black Hole Quencher) -HEG (Hexaethylglycol) -

<400>  1
ccccgaacgt cgaccgctcg gggcgatttc ggggatttta gggcgt                    46


<210>  2
<211>  21
<212>  DNA
<213>  Artificial

<220>
<223>  GSTP1 Scorpion Antisense Primer

<400>  2
aaaatcccgc gaactcccgc c                                               21


<210>  3
<211>  46
<212>  DNA
<213>  Artificial

<220>
<223>  GSTP1 Scorpion


<220>
<221>  modified_base
<222>  (23)..(23)
<223>  -BHQ-HEG-

<400>  3
cccgaacgtc gaccgctttc gggcgatttc ggggatttta gggcgt                    46


<210>  4
<211>  21
<212>  DNA
<213>  Artificial

<220>
<223>  GSTP1 Scorpion Antisense Primer

<400>  4
aaaatcccgc gaactcccgc c                                               21


<210>  5
<211>  44
<212>  DNA
<213>  Artificial

<220>
<223>  GSTP1 Scorpion


<220>
<221>  modified_base
<222>  (22)..(22)
<223>  -BHQ-HEG-

<400>  5
cggcgggagt tcgcgggcgc cgactaaatc acgacgccga ccgc                      44


<210>  6
<211>  22
<212>  DNA
<213>  Artificial

<220>
<223>  GSTP1 Scorpion Antisense Primer

<400>  6
cggttagttg cgcggcgatt tc                                              22


<210>  7
<211>  41
<212>  DNA
<213>  Artificial

<220>
<223>  GSTP1 Scorpion


<220>
<221>  modified_base
<222>  (19)..(19)
<223>  -BHQ-HEG-

<220>
<221>  modified_base
<222>  (25)..(25)
<223>  -BHQ-HEG-

<400>  7
cgggagttcg cgggtcccga ctaaatcacg acgccgaccg c                         41

<210>  8
<211>  22
<212>  DNA
<213>  Artificial

<220>
<223>  GSTP1 Scorpion Antisense Primer

<400>  8
cggttagttg cgcggcgatt tc                                                22


<210>  9
<211>  50
<212>  DNA
<213>  Artificial

<220>
<223>  GSTP1 Scorpion


<220>
<221>  modified_base
<222>  (27)..(27)
<223>  -BHQ-HEG-

<400>  9
gtggttgatg tttggggtat caaccacaat cccacaaact cccaccaacc                  50


<210>  10
<211>  27
<212>  DNA
<213>  Artificial

<220>
<223>  GSTP1 Scorpion Antisense Primer

<400>  10
gtggtgattt tggggatttt agggtgt                                           27


<210>  11
<211>  47
<212>  DNA
<213>  Artificial

<220>
<223>  GSTP1 Scorpion


<220>
<221>  modified_base
<222>  (24)..(24)
<223>  -BHQ-HEG-

<400>  11
accccagtgg ttgatgtttg gggtaatccc acaaactccc accaacc                     47


<210>  12
<211>  27
<212>  DNA
<213>  Artificial

<220>

<223>  GSTP1 Scorpion

<400>  12
gtggtgattt tggggatttt agggtgt                                          27


<210>  13
<211>  56
<212>  DNA
<213>  Artificial

<220>
<223>  GSTP1 Scorpion


<220>
<221>  modified_base
<222>  (28)..(28)
<223>  -BHQ-HEG-

<400>  13
ccccacaggt tggtgggagt ttgtggggcc caatactaaa tcacaacacc aaccac          56


<210>  14
<211>  27
<212>  DNA
<213>  Artificial

<220>
<223>  GSTP1 Scorpion Antisense Primer

<400>  14
tggttagttg tgtggtgatt ttgggga                                          27


<210>  15
<211>  46
<212>  DNA
<213>  Artificial

<220>
<223>  GSTP1 Scorpion


<220>
<221>  modified_base
<222>  (23)..(23)
<223>  -BHQ-HEG-

<400>  15
ccccgaacgt cgaccgctcg gggcgatttc ggggatttta gggcgt                     46


<210>  16
<211>  21
<212>  DNA
<213>  Artificial

<220>
<223>  GSTP1 Scorpion Antisense Primer

<400>  16
aaaatcccgc gaactcccgc c                                                21


<210>  17
<211>  53

<212>  DNA
<213>  Artificial

<220>
<223>  GSTP1 Scorpion


<220>
<221>  modified_base
<222>  (30)..(30)
<223>  -BHQ-HEG-

<400>  17
cgcacgccga acgtcgaccg caaacgtgcg cgatttcggg gattttaggg cgt          53


<210>  18
<211>  21
<212>  DNA
<213>  Artificial

<220>
<223>  GSTP1 Scorpion Antisense Primer

<400>  18
aaaatcccgc gaactcccgc c                                             21


<210>  19
<211>  47
<212>  DNA
<213>  Artificial

<220>
<223>  GSTP1 Scorpion


<220>
<221>  modified_base
<222>  (27)..(27)
<223>  -BHQ-HEG-

<400>  19
cgcacggcga actcccgccg acgtgcgtgt agcggtcgtc ggggttg                 47


<210>  20
<211>  22
<212>  DNA
<213>  Artificial

<220>
<223>  GSTP1 Scorpion Antisense Primer

<400>  20
gccccaatac taaatcacga cg                                            22


<210>  21
<211>  55
<212>  DNA
<213>  Artificial

<220>
<223>  GSTP1 Scorpion


<220>

<221>  modified_base
<222>  (35)..(35)
<223>  -BHQ-HEG-

<400>  21
ccgacgcaca aaaaaacacc ctaaaatccg tcggggttag ttgtgtggtg atttt          55


<210>  22
<211>  20
<212>  DNA
<213>  Artificial

<220>
<223>  GSTP1 Scorpion Antisense Primer

<400>  22
cacaacacca accactcttc                                                  20


<210>  23
<211>  29
<212>  DNA
<213>  Artificial

<220>
<223>  GSTP1 Taqman Primer

<400>  23
cgtgatttag tattggggcg gagcggggc                                        29


<210>  24
<211>  25
<212>  DNA
<213>  Artificial

<220>
<223>  GSTP1 Taqman Primer

<400>  24
atccccgaaa aacgaaccgc gcgta                                            25


<210>  25
<211>  26
<212>  DNA
<213>  Artificial

<220>
<223>  GSTP1 Taqman Probe

<400>  25
tcggaggtcg cgaggttttc gttgga                                           26


<210>  26
<211>  48
<212>  DNA
<213>  Artifical


<220>
<221>  misc_feature
<223>  GSTP1 SCORPION

<220>
<221>  modified_base

<222>  (27)..(27)
<223>  -BHQ-HEG-

<400>  26
cggccctaaa accgctacga gggccggaag cgggtgtgta agtttcgg                48


<210>  27
<211>  26
<212>  DNA
<213>  Artifical


<220>
<221>  misc_feature
<223>  GSTP1 SCORPION Antisense Primer

<400>  27
acgaaatata cgcaacgaac taacgc                                        26


<210>  28
<211>  44
<212>  DNA
<213>  Artificial

<220>
<223>  GSTP1 SCORPION


<220>
<221>  misc_feature
<223>  GSTPI SCORPION

<220>
<221>  modified_base
<222>  (24)..(24)
<223>  -BHQ-HEG-

<400>  28
ccggtcgcga ggttttcgac cggccgaaaa acgaaccgcg cgta                    44


<210>  29
<211>  27
<212>  DNA
<213>  Artificial

<220>
<223>  GSTP1 SCORPION


<220>
<221>  misc_feature
<223>  GSTP1 SCORPION Antisense Primer

<400>  29
gggcgggatt atttttataa ggttcgg                                       27


<210>  30
<211>  49
<212>  DNA
<213>  Artificial

<220>
<223>  RASSFIA Scorpion

<220>
<221>  modified_base
<222>  (25)..(25)
<223>  -BHQ-HEG-

<400>  30
gccgcggttt cgttcggttc gcggccccgt acttcgctaa ctttaaacg                49


<210>  31
<211>  18
<212>  DNA
<213>  Artificial

<220>
<223>  RASSFIA Scorpion Antisense Primer

<400>  31
gcgttgaagt cggggttc                                                  18


<210>  32
<211>  47
<212>  DNA
<213>  Artifical


<220>
<221>  misc_feature
<223>  RARB2 SCORPION

<220>
<221>  modified_base
<222>  (27)..(27)
<223>  -BHQ-HEG-

<400>  32
cggcgcccga cgatacccaa agcgccgaac gcgagcgatt cgagtag                  47


<210>  33
<211>  24
<212>  DNA
<213>  Artificial

<220>
<223>  RARB2 SCORPION


<220>
<221>  misc_feature
<223>  RARB2 SCORPION Antisense Primer

<400>  33
cttacaaaaa accttccgaa tacg                                           24


<210>  34
<211>  44
<212>  DNA
<213>  Artificial

<220>
<223>  APC Scorpion


<220>

<221>  modified_base
<222>  (25)..(25)
<223>  -BHQ-HEG-

<400>  34
gccggcgggt tttcgacggg ccggccgaac caaaacgctc ccca                44


<210>  35
<211>  25
<212>  DNA
<213>  Artificial

<220>
<223>  APC Scorpion Antisense Primer

<400>  35
gtcggttacg tgcgtttata tttag                                     25


<210>  36
<211>  44
<212>  DNA
<213>  Artificial

<220>
<223>  Actin Scorpion


<220>
<221>  modified_base
<222>  (22)..(22)
<223>  -BHQ-HEG-

<400>  36
gcgcccaacc gcacaagggc gcgggtatat tttcgagggg tacg                44


<210>  37
<211>  18
<212>  DNA
<213>  Artificial

<220>
<223>  ACTIN Scorpion Antisense Primer

<400>  37
cgacccgcac tccgcaat                                             18


<210>  38
<211>  52
<212>  DNA
<213>  Artificial

<220>
<223>  ACTIN Scorpion


<220>
<221>  modified_base
<222>  (27)..(27)
<223>  -BHQ-HEG-

<400>  38
ccgcgcatca ccaccccaca cgcgcgggga gtatataggt tggggaagtt tg       52

<210>  39
<211>  27
<212>  DNA
<213>  Artificial

<220>
<223>  ACTIN Scorpion Antisense Primer

<400>  39
aacacacaat aacaaacaca aattcac                                          27


<210>  40
<211>  50
<212>  DNA
<213>  Artificial

<220>
<223>  ACTIN Scorpion


<220>
<221>  modified_base
<222>  (27)..(27)
<223>  -BHQ-HEG-

<400>  40
cccggctaaa cccaccatcc agccgggggg agggtagttt agttgtggtt                 50


<210>  41
<211>  29
<212>  DNA
<213>  Artificial

<220>
<223>  ACTIN Scorpion Antisense Primer

<400>  41
caaaacaaaa aaactaaatc tacacaacc                                        29


<210>  42
<211>  47
<212>  DNA
<213>  Artificial

<220>
<223>  ACTIN Scorpion


<220>
<221>  modified_base
<222>  (26)..(26)
<223>  -BHQ-HEG-

<400>  42
ccgcggaaca ttcaactcaa ccgcggggag gaggaaggta ggttttt                    47


<210>  43
<211>  29
<212>  DNA
<213>  Artificial

<220>
<223>  ACTIN Scorpion Antisense Primer

<400>  43
acatacaaca atcaataaca taaaaccac                                          29


<210>  44
<211>  47
<212>  DNA
<213>  Artificial

<220>
<223>  PTGS2/COX2 Scorpion


<220>
<221>  modified_base
<222>  (22)..(22)
<223>  -BHQ-HEG-

<400>  44
cacgccgccg tatctaggcg tggtttgttt cgacgtgatt ttttcga                      47


<210>  45
<211>  21
<212>  DNA
<213>  Artificial

<220>
<223>  PTGS2/COX2 Antisense Primer

<400>  45
gcaaaaaatc ccctctcccg c                                                  21


<210>  46
<211>  45
<212>  DNA
<213>  Artificial

<220>
<223>  PTGS2/COX2 Scorpion


<220>
<221>  modified_base
<222>  (22)..(22)
<223>  -BHQ-HEG-

<400>  46
gccgcgcaca aatttccgcg gcgaattggt tttcggaagc gttcg                        45


<210>  47
<211>  16
<212>  DNA
<213>  Artificial

<220>
<223>  PTGS2/COX2 Antisense Primer

<400>  47
cccgaattcc accgcc                                                        16


<210>  48
<211>  46
<212>  DNA
<213>  Artificial

<220>
<223>  PTGS2/COX2 Scorpion


<220>
<221>  modified_base
<222>  (23)..(23)
<223>  -BHQ-HEG-

<400>  48
ggcggaacgc acaaatttcc gccgaattgg ttttcggaag cgttcg                    46


<210>  49
<211>  16
<212>  DNA
<213>  Artificial

<220>
<223>  PTGS2/COX2 Antisense Primer

<400>  49
cccgaattcc accgcc                                                     16


<210>  50
<211>  51
<212>  DNA
<213>  Artificial

<220>
<223>  PTGS2/COX2 Scorpion


<220>
<221>  modified_base
<222>  (30)..(30)
<223>  -BHQ-HEG-

<400>  50
tgccgccgcc gtatctaatg gcggcagttt gtttcgacgt gattttttcg a             51


<210>  51
<211>  21
<212>  DNA
<213>  Artificial

<220>
<223>  PTGS2/COX2 Antisense Primer


<220>
<221>  misc_feature
<223>  PTGS2/COX2 Antisense Primer

<400>  51
gcaaaaaatc ccctctcccg c                                               21


<210>  52
<211>  39
<212>  DNA
<213>  Artificial

<220>
<223>  CDHI SCORPION

<220>
<221>  modified_base
<222>  (19)..(19)
<223>  -BHQ-HEG-

<400>  52
cgccgaatac gatcggcggt tcgttttagt tcggttcga                    39


<210>  53
<211>  18
<212>  DNA
<213>  Artificial

<220>
<223>  CDH1 SCORPION Antisense Primer

<400>  53
accgaaaacg ccgaacga                                           18


<210>  54
<211>  37
<212>  DNA
<213>  Artificial

<220>
<223>  15LO1 SCORPION


<220>
<221>  modified_base
<222>  (19)..(19)
<223>  -HEG-BHQ-

<400>  54
ggcggcgttc gggccgcccc gtacgaacca caatcgc                      37


<210>  55
<211>  21
<212>  DNA
<213>  Artificial

<220>
<223>  15LO1 SCORPION Antisense Primer

<400>  55
ggggtttcgt tttatgtcgg t                                       21


<210>  56
<211>  2671
<212>  DNA
<213>  Homo sapiens


<220>
<221>  misc_feature
<223>  15LO1       >gi|187190|gb|M23892.1|HUMLOX15A Human 15-lipoxygenase mRNA,
        complete cds

<400>  56
aagatgggtc tctaccgcat ccgcgtgtcc actggggcct cgctctatgc cggttccaac      60

aaccaggtgc agctgtggct ggtcggccag cacggggagg cggcgctcgg gaagcgactg     120

tggcccgcac ggggcaagga gacagaactc aaggtggaag taccggagta tctggggccg 180
ctgctgtttg tgaaactgcg caaacggcac ctccttaagg acgacgcctg gttctgcaac 240
tggatctctg tgcagggccc cggagccggg gacgaggtca ggttcccttg ttaccgctgg 300
gtggagggca acggcgtcct gagcctgcct gaaggcaccg gccgcactgt gggcgaggac 360
cctcagggcc tgttccagaa acaccgggaa gaagagctgg aagagagaag gaagttgtac 420
cggtggggaa actggaagga cgggttaatt ctgaatatgg ctggggccaa actatatgac 480
ctccctgtgg atgagcgatt tctggaagac aagagagttg actttgaggt ttcgctggcc 540
aaggggctgg ccgacctcgc tatcaaagac tctctaaatg ttctgacttg ctggaaggat 600
ctagatgact tcaaccggat tttctggtgt ggtcagagca agctggctga gcgcgtgcgg 660
gactcctgga aggaagatgc cttatttggg taccagtttc ttaatggcgc caaccccgtg 720
gtgctgaggc gctctgctca ccttcctgct cgcctagtgt tccctccagg catggaggaa 780
ctgcaggccc agctggagaa ggagctggag ggaggcacac tgttcgaagc tgacttctcc 840
ctgctggatg ggatcaaggc caacgtcatt ctctgtagcc agcagcacct ggctgcccct 900
ctagtcatgc tgaaattgca gcctgatggg aaactcttgc ccatggtcat ccagctccag 960
ctgccccgca caggatcccc accacctccc cttttcttgc ctacggatcc cccaatggcc 1020
tggcttctgg ccaaatgctg ggtgcgcagc tctgacttcc agctccatga gctgcagtct 1080
catcttctga ggggacactt gatggctgag gtcattgttg tggccaccat gaggtgcctg 1140
ccgtcgatac atcctatctt caagcttata attccccacc tgcgatacac cctggaaatt 1200
aacgtccggg ccaggactgg gctggtctct gacatgggaa ttttcgacca gataatgagc 1260
actggtgggg gaggccacgt gcagctgctc aagcaagctg gagccttcct aacctacagc 1320
tccttctgtc cccctgatga cttggccgac cgggggctcc tgggagtgaa gtcttccttc 1380
tatgcccaag atgcgctgcg gctctgggaa atcatctatc ggtatgtgga aggaatcgtg 1440
agtctccact ataagacaga cgtggctgtg aaagacgacc cagagctgca gacctggtgt 1500
cgagagatca ctgaaatcgg gctgcaaggg gcccaggacc gagggtttcc tgtctcttta 1560
caggctcggg accaggtttg ccactttgtc accatgtgta tcttcacctg caccggccaa 1620
cacgcctctg tgcacctggg ccagctggac tggtactctt gggtgcctaa tgcaccctgc 1680
acgatgcggc tgcccccgcc aaccaccaag gatgcaacgc tggagacagt gatggcgaca 1740
ctgcccaact tccaccaggc ttctctccag atgtccatca cttggcagct gggcagacgc 1800
cagcccgtta tggtggctgt gggccagcat gaggaggagt attttcgggc cctgagcct 1860
aaggctgtgc tgaagaagtt cagggaggag ctggctgccc tggataagga aattgagatc 1920
cggaatgcaa agctggacat gccctacgag tacctgcggc ccagcgtggt ggaaaacagt 1980
gtggccatct aagcgtcgcc accctttggt tatttcagcc cccatcaccc aagccacaag 2040
ctgacccctt cgtggttata gccctgccct cccaagtccc accctcttcc catgtcccac 2100
cctccctaga ggggcacctt ttcatggtct ctgcacccag tgaacacatt ttactctaga 2160

ggcatcacct gggaccttac tcctctttcc ttccttcctc ctttcctatc ttccttcctc 2220

tctctcttcc tctttcttca ttcagatcta tatggcaaat agccacaatt atataaatca 2280

tttcaagact agaatagggg gatataatac atattactcc acacctttta tgaatcaaat 2340

atgatttttt tgttgttgtt aagacagagt ctcactttga cacccaggct ggagtgcagt 2400

ggtgccatca ccacggctca ctgcagcctc agcgtcctgg gctcaaatga tcctcccacc 2460

tcagcctcct gagtagctgg gactacaggc tcatgccatc atgcccagct aatatttttt 2520

tattttcgtg gagacggggc ctcactatgt tgcctaggct ggaaatagga ttttgaaccc 2580

aaattgagtt taacaataat aaaaagttgt tttacgctaa agatggaaaa gaactaggac 2640

tgaactattt taaataaaat attggcaaaa g 2671

<210> 57
<211> 1193
<212> DNA
<213> Homo sapiens

<220>
<221> misc_feature
<223> CDH1 >gi|509604|gb|L34545.1|HUMECADN Human E-cadherin gene, promoter
region and 5' end

<400> 57
   tctagaaaaa ttttttaaaa aattaggccg ctcgagcgag agtgcagtgg ctcacgcctg 60
   taatccaaca cttcaggagg ctgaagaggg tggatcacct gaggtcagga gttccagacc 120
   agcctggcca acatggtgaa accccgtctt gtactaaaaa tacaaaatta gccggtgtgg 180
   tggcacacgc ctgtagtccc agctactcaa taggctgaga caggagagtc tcttgaaccc 240
   ggcaggcgga ggttgcagtg agccgagatc gtgccactgc actccagcct gggcaagaca 300
   gagcgagact ccgtctcaaa aaatacaaac aaaacaaaca aacaaaaaat taggctgcta 360
   gctcagtggc tcatggctca cacctgaaat cctagcactt tgggaggcca aggcaggagg 420
   atcgcttcag cccaggagtt cgagaccagg ctgggcaata cagggagaca cagcgccccc 480
   actgcccctg tccgccccga cttgtctctc tacaaaaagg caaaagaaaa aaaaaattag 540
   cctggcgtgg tggtgtgcac ctgtactccc agctactaga gaggctgggg ccagaggacc 600
   gcttgagccc aggagttcga ggctgcagtg agctgtgatc gcaccactgc actccagctt 660
   gggtgaaaga gtgagcccca tctccaaaac gaacaaacaa aaaatcccaa aaaacaaaag 720
   aactcagcca agtgtaaaag ccctttctga tcccaggtct tagtgagcca ccggcggggc 780
   tgggattcga acccagtgga atcagaaccg tgcaggtccc ataacccacc tagaccctag 840
   caactccagg ctagagggtc accgcgtcta tgcgaggccg ggtgggcggg ccgtcagctc 900
   cgccctgggg aggggtccgc gctgctgatt ggctgtggcc ggcaggtgaa ccctcagcca 960
   atcagcggta cggggggcgg tgctccgggg ctcacctggc tgcagccacg caccccctct 1020
   cagtggcgtc ggaactgcaa agcacctgtg agcttgcgga agtcagttca gactccagcc 1080
   cgctccagcc cggcccgacc cgaccgcacc cggcgcctgc cctcgctcgg cgtccccggc 1140
   cagccatggg cccttggagc cgcagcctct cggcgctgct gctgctgctg cag 1193

<210> 58
<211> 2635
<212> DNA
<213> Homo sapiens

<220>
<221> misc_feature
<223> Hin-1 REGION ON CHROMOSOME 5( Alternate name: (SCGB3A1)

<220>
<221> misc_feature
<223> This gene can be found on Chromosome 5 at location
179,949,712-179,951,146. The start of this gene is located in
Contig AC122714.2.1.190024.
>chromosome:NCBI35:5:179949112:179951746:-1

<400> 58
atctgtcata cttctgcagg tgacagacga gtgaggacat ttagagaaac tcaggaacaa 60

accaggccca tccactggcg tcgaggctag tttcgaagac agaaaaacgc cccacttctg 120

tttgcactgt ggctgcctgt ggcccggccg gggaggcggc cgggagtgag gcctgatcgt 180

ccctggcgcc tccacctccc caggcgcaga aggcgcccac gaggaccccc agtgcccgac 240

gttgccacgg tctgggatca gaggcaggga ccagggagcc aggaactgcg ccgcccccgc 300

ccctgccctg gcgcgaggga agctcccctc acccgggccc agccctgcag gggggcgcgt 360

ggggtcagac cgcaaagcga aggtgcgggc cggggtgggc ctcgcggaga caaaggccgg 420

gcctgcctgc tctcagaggg ccccagcgcc tgccaagagg aagtcctcga ggcccgggca 480

gggaaggggg cacgggcttc ccagggcccg ccggccgcag caggaagttg gccagggcac 540

ggccgtgagc ggagcgggca gggctttctc aggagcgcgg gcgaggccgg cgctggaggg 600

gcgaggaccg ggtataagaa gcctcgtggc cttgcccggg cagccgcagg ttccccgcgc 660

gccccgagcc cccgcgccat gaagctcgcc gccctcctgg ggctctgcgt ggccctgtcc 720

tgcagctccg gtgagcgccc cgggctcctg gcgcgcacgg tggggcctga ggcctcggcg 780

cccgctgcgc ccccgccgct gcctgcgccg atcgtggatc ccaggtcctt ccagcctcgc 840

ccggcgccca gagggctccg ccaccccggg gccccgcgcc tgcagcccgc ggcctccccc 900

tctcagggct gtctccagcc tcgtgccggg atggaggccg cccctggcct ggggacaccc 960

gtctgccccc cgtctggaga ccggctcctc atcctgcaag gcgcagcgcg agtgtcccct 1020

cccttggggg cctgtcccgg accctgcaca gagttcaccg gtccttccgc caccctcagg 1080

ccactccggt gaccctgcag cgtctcctgg cggggccgcc tccccagacc cgcctgtgtc 1140

ccgggggctc gcacctggca ggcctcggcg caggagggag gggcggtcgg ggagccgggc 1200

agggcgcgac ggttcctggg cgcctcccgc gggggcgcgt cctggacctg ccttctgggg 1260

accccggcgc gcaggcggtg acccctccct gttcgcttgc agctgctgct ttcttagtgg 1320

gctcggccaa gcctgtggcc cagcctgtcg ctgcgctgga gtcggcggcg gaggccgggg 1380

ccgggaccct ggccaacccc ctcggcaccc tcaacccgct gaagctcctg ctgagcagcc 1440

tgggcatccc cgtgaaccac ctcatagagg gctcccagaa gtgtgtggct gagctgggtc 1500

cccaggccgt gggggccgtg aaggccctga aggccctgct ggtaaagtgg gcaccccggg 1560

tgcccttcct gcgcgggcat cccttcccgg ccagcctcaa aactgcacca gaggtgtccc 1620

ggccctacgt cagggctgtt tcccgtcctg cccctccaag ccctgtccct gggagaagcc 1680

cgggtctccg ggtgggaact gggtggggcg catccgagct gcaggggcgg ggaaggcgga 1740

gatggccccg cgcgggtgcg cgtcccccgg agacgccagc ggaaccgccc cgctcccgct 1800

ccccacagag ccggccgctg ccgcgccccc gcctgagttc ttggtggcag ggctgggggc 1860

actcaagctc ggcttctgct ttccaggggg ccctgacagt gtttggctga gccgagactg 1920

gagcatctac acctgaggac aagacgctgc ccacccgcga gggctgaaaa ccccgccgcg 1980

gggaggaccg tccatcccct tcccccggcc cctctcaata aacgtggtta agagcagctc 2040

gagcctggta ttttctcatc caaatgctga tccctgtggg ggaccgagag tgccaagtcc 2100

cagattttgt gaggggtgct gcgtgagaga gggagagaga ggccgcgaga gtctgcaggt 2160

ggcatagagc cagcagtgca aggtgacaga gatggggcca gtatcagagg taaaccggat 2220

ttggaaacaa aactggcagg tctttcttcc tgttgtgctc tgtaactgtt tatgaagcat 2280

aaaaattatc tactccttga agactggcga gaatgcaaac gcaccaaagc catttgttgc 2340

taaaatttga aagtttttat tttttccctc tatacttgta gatttcttcc tcggaatggg 2400

ggaggaatga gggcttcagg aaatccgctg ttgggcttgt tttccctgca gtttctgctg 2460

gttttgtcca aataacctag tgactttgat tccttctact ctagcggaga gaccaccttt 2520

tgtcaactga tctagtgttg gtgggaaggt gcccagggaa cccagacatc ggtgtccctt 2580

tcaggggaag cctcccagtt gacttccttg ctgggatgaa taactctgcc tcagg 2635

<210> 59
<211> 4260
<212> DNA
<213> Homo sapiens

<220>
<221> misc_feature
<223> >gi|341173|gb|M24485.1|HUMGSTP1G Homo sapiens (clone pHGST-pi)
glutathione S-transferase pi (GSTP1) gene, complete cds

<400> 59
aacaagagat caatatctag aataaatgga gatctgcaaa tcaacagaaa gtaggcagca 60

aagccaaaga aaatagccta aggcacagcc actaaaagga acgtgatcat gtcctttgca 120

gggacatggg tggagctgga agccgttagc ctcagcaaac tcacacagga acagaaaacc 180

agcgagaccg catggtctca cttataagtg ggagctgaac aatgagaaca catggtcaca 240

tggcggcgat caacacacac tggtgcctgt tgagcggggt gctggggagg gagagtacca 300

ggaagaatag ctaagggata ctgggcttaa tacctgggtg atgggatgat ctgtacagca 360

aaccatcatg gcgcacacac ctatgtaaca aacctgcaca tcctgcacat gtaccccaga 420
acttcaaata aaagttggac ggccaggcgt ggtggctcac gcctgtaatc ccagcacttt 480
gggaagccga ggcgtgcaga tcacctaagg tcaggagttc gagaccagcc cggccaacat 540
ggtgaaaccc cgtctctact aaaaatacaa aaatcagcca gatgtggcac gcacctataa 600
ttccacctac tcgggaggct gaagcagaat tgcttgaacc cgagaggcgg aggttgcagt 660
gagccgccga gatcgcgcca ctgcactcca gcctgggcca cagcgtgaga ctacgtcata 720
aaataaaata aaataacaca aaataaaata aaataaaata aaataaaata aaataataaa 780
ataaaataaa ataaaataaa ataaaataaa ataaagcaat ttcctttcct ctaagcggcc 840
tccacccctc tcccctgccc tgtgaagcgg gtgtgcaagc tccgggatcg cagcggtctt 900
agggaatttc cccccgcgat gtcccggcgc gccagttcgc tgcgcacact tcgctgcggt 960
cctcttcctg ctgtctgttt actccctagg ccccgctggg gacctgggaa agagggaaag 1020
gcttccccgg ccagctgcgc ggcgactccg gggactccag ggcgcccctc tgcggccgac 1080
gcccggggtg cagcggccgc cggggctggg gccggcggga gtccgcggga ccctccagaa 1140
gagcggccgg cgccgtgact cagcactggg gcggagcggg gcgggaccac ccttataagg 1200
ctcggaggcc gcgaggcctt cgctggagtt tcgccgccgc agtcttcgcc accagtgagt 1260
acgcgcggcc cgctccccgg ggatggggct cagagctccc agcatggggc caacccgcag 1320
catcaggccc gggctcccgg cagggctcct cgcccacctc gagacccggg acgggggcct 1380
aggggaccca ggacgtcccc agtgccgtta gcggctttca gggggcccgg agcgcctcgg 1440
ggagggatgg gaccccgggg gcggggaggg ggggcaggct gcgctcaccg cgccttggca 1500
tcctcccccg ggctccagca aacttttctt tgttcgctgc agtgccgccc tacaccgtgg 1560
tctatttccc agttcgaggt aggagcatgt gtctggcagg gaagggaggc aggggctggg 1620
gctgcagccc acagcccctc gcccacccgg agagatccga acccccttat ccctccgtcg 1680
tgtggctttt accccgggcc tccttcctgt tccccgcctc tcccgccatg cctgctcccc 1740
gccccagtgt tgtgtgaaat cttcggagga acctgtttac ctgttccctc cctgcactcc 1800
tgacccctcc ccgggttgct gcgaggcgga gtcggcccgg tccccacatc tcgtacttct 1860
ccctccccgc aggccgctgc gcggccctgc gcatgctgct ggcagatcag ggccagagct 1920
ggaaggagga ggtggtgacc gtggagacgt ggcaggaggg ctcactcaaa gcctcctgcg 1980
taagtgacca tgcccgggca aggggagggg gtgctgggcc ttagggggct gtgactagga 2040
tcgggggacg cccaagctca gtgcccctcc ctgagccatg cctcccccaa cagctatacg 2100
ggcagctccc caagttccag gacggagacc tcaccctgta ccagtccaat accatcctgc 2160
gtcacctggg ccgcaccctt ggtgagtctt gaacctccaa gtccagggca ggcatgggca 2220
agcctctgcc cccggagccc ttttgtttaa atcagctgcc ccgcagccct ctggagtgga 2280
ggaaactgag acccactgag gttacgtagt ttgcccaagg tcaagcctgg gtgcctgcaa 2340
tccttgccct gtgccaggct gcctcccagg tgtcaggtga gctctgagca cctgctgtgt 2400

ggcagtctct catccttcca cgcacatcct cttcccctcc tcccaggctg gggctcacag 2460

acagccccct ggttggccca tccccagtga ctgtgtgttg atcaggcgcc cagtcacgcg 2520

gcctgctccc ctccacccaa ccccagggct ctatgggaag gaccagcagg aggcagccct 2580

ggtggacatg gtgaatgacg gcgtggagga cctccgctgc aaatacatct ccctcatcta 2640

caccaactat gtgagcatct gcaccagggt tgggcactgg gggctgaaca aagaaagggg 2700

cttcttgtgc cctcaccccc cttacccctc aggtggcttg ggctgacccc ttcttgggtc 2760

agggtgcagg ggctgggtca gctctgggcc aggggcccag gggcctggga caagacacaa 2820

cctgcaccct tattgcctgg gacatcaacc agccaagtaa cgggtcatgg gggcgagtgc 2880

aaggacagag acctccagca actggtggtt tctgatctcc tggggtggcg agggcttcct 2940

ggagtagcca gaggtggagg aggatttgtc gccagtttct ggatggaggt gctggcactt 3000

ttagctgagg aaaatatgca gacacagagc acatttgggg acctgggacc agttcagcag 3060

aggcagcgtg tgtgcgcgtg cgtgtgcgtg tgtgtgcgtg tgtgtgtgta cgcttgcatt 3120

tgtgtcgggt gggtaaggag atagagatgg gcgggcagta ggcccaggtc ccgaaggcct 3180

tgaacccact ggtttggagt ctcctaaggg caatgggggc cattgagaag tctgaacagg 3240

gctgtgtctg aatgtgaggt ctagaaggat cctccagaga agccagctct aaagcttttg 3300

caatcatctg gtgagagaac ccagcaagga tggacaggca gaatggaata gagatgagtt 3360

ggcagctgaa gtggacagga tttggtacta gcctggttgt ggggagcaag cagaggagaa 3420

tctgggactc tggtgtctgg cctggggcag acgggggtgt ctcaggggct gggagggatg 3480

agagtaggat gatacatggt ggtgtctggc aggaggcggg caaggatgac tatgtgaagg 3540

cactgcccgg gcaactgaag ccttttgaga ccctgctgtc ccagaaccag ggaggcaaga 3600

ccttcattgt gggagaccag gtgagcatct ggccccatgc tgttccttcc tcgccaccct 3660

ctgcttccag atggacacag gtgtgagcca tttgtttagc aaagcagagc agacctaggg 3720

gatgggctta ggccctctgc ccccaattcc tccagcctgc tcccgctggc tgagtcccta 3780

gcccccctgc cctgcagatc tccttcgctg actacaacct gctggacttg ctgctgatcc 3840

atgaggtcct agcccctggc tgcctggatg cgttccccct gctctcagca tatgtggggc 3900

gcctcagtgc ccggcccaag ctcaaggcct tcctggcctc ccctgagtac gtgaacctcc 3960

ccatcaatgg caacgggaaa cagtgagggt tggggggact ctgagcggga ggcagagttt 4020

gccttccttt ctccaggacc aataaaattt ctaagagagc tactatgagc actgtgtttc 4080

ctgggacggg gcttaggggt tctcagcctc gaggtcggtg ggagggcaga gcagaggact 4140

agaaaacagc tcctccagca cagtcagtgg cttcctggag ccctcagcct ggctgtgttt 4200

actgaacctc acaaactaga agaggaagaa aaaaaaagag agagagaaac aaagagaaat 4260

<210> 60
<211> 2011
<212> DNA
<213> Homo sapiens

<220>
<221>  misc_feature
<223>  B-Actin      >gi|28337|emb|Y00474.1|HSACTBPR Human beta-actin gene
5'-flanking
       region, CpG Island 1656 to 1955

<400>  60
gagctctgtc tcttggccag ctgaatggag gcccagcggc aacacaggtc ctgcctgggg      60

atcaggtctg ctctgcaccc caccttgctg cctggagccg cccacctgac aacctctcat     120

ccctgctctg tagatccggt cccatcccca ctgcccaccc caccccccca gcactccacc     180

cagttcaacg ttccacgaac ccccagaacc agccctcatc aacaggcagc aagaagggcc     240

ccccgcccat cgccccacaa cgccagccgg gtgaactgta gcgttggcag gtcctgaggc     300

agctgaaaga tacaaggcca gggacaggac agtcccatcc ccaggaggca gggagtatac     360

aggctgggga agtttgccct tgcgtggggt ggtgatggag gaggctcagc aagtcttctg     420

gactgtgaac ctgtgtctgc cactgtgtgc tgggtggtgg tcatctttcc caccaggctg     480

tggcctctgc aaccttcaag ggaggagcag gtcccattgg ctgagcacag ccttgtacgt     540

gaactgaaca agcagcctcc ttcctggcca caggttccat gtccttatat ggactcatct     600

ttgcctattg cgacacacac tcaatgaaca cctactacgc gctgcaaaga gccccgcagg     660

cctgaggtgc ccccacctca ccactcttcc tatttttgtg taaaaatcca gcttcttgtc     720

accacctcca aggaggggga ggaggaggaa ggcaggttcc tctaggctga gccgaatgcc     780

cctctgtggt cccacgccac tgatcgctgc atgcccacca cctgggtaca cacagtctgt     840

gattcccgga gcagaacgga ccctgcccac ccggtcttgt gtgctactca gtggacagac     900

ccaaggcaag aaagggtgac aaggacaggg tcttcccagg ctggctttga gttcctagca     960

ccgccccgcc cccaatcctc tgtggcacat ggagtcttgg tccccagagt cccccagcgg    1020

cctccagatg gtctgggagg gcagttcagc tgtggctgcg catagcagac atacaacgga    1080

cggtgggccc agacccaggc tgtgtagacc cagccccccc gccccgcagt gcctaggtca    1140

cccactaacg ccccaggcct ggtcttggct gggcgtgact gttaccctca aaagcaggca    1200

gctccagggt aaaaggtgcc ctgccctgta gagcccactt ccttcccagg gctgcggctg    1260

ggtaggtttg tagccttcat cacgggccac ctccagccac tggaccgctg gcccctgccc    1320

tgtcctgggg agtgtggtcc tgcgactcta atggccgcaa gccacctgac tcccccaaca    1380

ccacactcta cctctcaagc ccaggtctct ccctagtgac ccacccagca catttagcta    1440

gctgagcccc acagccagag gtcctcaggc cctgctttca gggcagttgc tctgaagtcg    1500

gcaaggggga gtgactgcct ggccactcca tgccctccaa gagctccttc tgcaggagcg    1560

tacagaaccc agggccctgg cacccgtgca gaccctggcc caccccacct gggcgctcag    1620

tgcccaagag atgtccacac ctaggatgtc ccgcggtggg tggggggccc gagagacggg    1680

caggccgggg gcaggcctgg ccatgcgggg ccgaaccggg cactgcccag cgtggggcgc    1740

gggggccacg gcgcgcgccc ccagcccccg ggcccagcac cccaaggcgg ccaacgccaa    1800

aactctccct cctcctcttc ctcaatctcg ctctcgctct tttttttttt cgcaaaagga 1860

ggggagaggg ggtaaaaaaa tgctgcactg tcggcgaagc cggtgagtga gcggcgcggg 1920

gccaatcgcg tgcgccgttc cgaaagttgc cttttatggc tcgagcggcc gcggcggcgc 1980

cctataaaac ccagcggcgc gacgcgccac c 2011

<210> 61
<211> 1792
<212> DNA
<213> Homo sapiens

<220>
<221> misc_feature
<223> >gi|5016088|ref|NM_001101.2| Homo sapiens actin, beta (ACTB), mRNA

<400> 61
cgcgtccgcc ccgcgagcac agagcctcgc ctttgccgat ccgccgcccg tccacacccg 60

ccgccagctc accatggatg atgatatcgc cgcgctcgtc gtcgacaacg gctccggcat 120

gtgcaaggcc ggcttcgcgg gcgacgatgc cccccgggcc gtcttcccct ccatcgtggg 180

gcgccccagg caccagggcg tgatggtggg catgggtcag aaggattcct atgtgggcga 240

cgaggcccag agcaagagag gcatcctcac cctgaagtac cccatcgagc acggcatcgt 300

caccaactgg gacgacatgg agaaaatctg gcaccacacc ttctacaatg agctgcgtgt 360

ggctcccgag gagcaccccg tgctgctgac cgaggccccc ctgaacccca aggccaaccg 420

cgagaagatg acccagatca tgtttgagac cttcaacacc ccagccatgt acgttgctat 480

ccaggctgtg ctatccctgt acgcctctgg ccgtaccact ggcatcgtga tggactccgg 540

tgacggggtc acccacactg tgcccatcta cgaggggtat gccctccccc atgccatcct 600

gcgtctggac ctggctggcc gggacctgac tgactacctc atgaagatcc tcaccgagcg 660

cggctacagc ttcaccacca cggccgagcg ggaaatcgtg cgtgacatta aggagaagct 720

gtgctacgtc gccctggact tcgagcaaga gatggccacg gctgcttcca gctcctccct 780

ggagaagagc tacgagctgc ctgacggcca ggtcatcacc attggcaatg agcggttccg 840

ctgccctgag gcactcttcc agccttcctt cctgggcatg gagtcctgtg gcatccacga 900

aactaccttc aactccatca tgaagtgtga cgtggacatc cgcaaagacc tgtacgccaa 960

cacagtgctg tctggcggca ccaccatgta ccctggcatt gccgacagga tgcagaagga 1020

gatcactgcc ctggcaccca gcacaatgaa gatcaagatc attgctcctc ctgagcgcaa 1080

gtactccgtg tggatcggcg gctccatcct ggcctcgctg tccaccttcc agcagatgtg 1140

gatcagcaag caggagtatg acgagtccgg cccctccatc gtccaccgca aatgcttcta 1200

ggcggactat gacttagttg cgttacaccc tttcttgaca aaacctaact tgcgcagaaa 1260

acaagatgag attggcatgg ctttatttgt tttttttgtt ttgttttggt tttttttttt 1320

tttttggctt gactcaggat ttaaaaactg gaacggtgaa ggtgacagca gtcggttgga 1380

gcgagcatcc cccaaagttc acaatgtggc cgaggacttt gattgcacat tgttgttttt 1440

ttaatagtca ttccaaatat gagatgcatt gttacaggaa gtcccttgcc atcctaaaag 1500

ccaccccact tctctctaag gagaatggcc cagtcctctc ccaagtccac acaggggagg 1560

tgatagcatt gctttcgtgt aaattatgta atgcaaaatt tttttaatct tcgccttaat 1620

acttttttat tttgttttat tttgaatgat gagccttcgt gccccccctt cccccttttt 1680

gtcccccaac ttgagatgta tgaaggcttt tggtctccct gggagtgggt ggaggcagcc 1740

agggcttacc tgtacactga cttgagacca gttgaataaa agtgcacacc tt 1792

<210> 62
<211> 2762
<212> DNA
<213> Homo sapiens

<220>
<221> misc_feature
<223> RARB2 >gi|14916495|ref|NM_016152.2| Homo sapiens retinoic acid
 receptor, beta (RARB), transcript variant 2, mRNA

<400> 62
gtgacagaag tagtaggaag tgagctgttc agaggcagga gggtctattc tttgccaaag 60

gggggaccag aattccccat gcgagctgtt tgaggactgg gatgccgaga acgcgagcga 120

tccgagcagg gtttgtctgg gcaccgtcgg ggtaggatcc ggaacgcatt cggaaggctt 180

tttgcaagca tttacttgga aggagaactt gggatctttc tgggaacccc ccgccccggc 240

tggattggcc gagcaagcct ggaaaatgca attgaaacac agagcaccag ctctgaggaa 300

ctcgtcccaa gccccccatc tccacttcct ccccctcgag tgtacaaacc ctgcttcgtc 360

tgccaggaca aatcatcagg gtaccactat ggggtcagcg cctgtgaggg atgtaagggc 420

tttttccgca gaagtattca gaagaatatg atttacactt gtcaccgaga taagaactgt 480

gttattaata aagtcaccag gaatcgatgc caatactgtc gactccagaa gtgctttgaa 540

gtgggaatgt ccaaagaatc tgtcaggaat gacaggaaca agaaaaagaa ggagacttcg 600

aagcaagaat gcacagagag ctatgaaatg acagctgagt tggacgatct cacagagaag 660

atccgaaaag ctcaccagga aactttccct tcactctgcc agctgggtaa atacaccacg 720

aattccagtg ctgaccatcg agtccgactg gacctgggcc tctgggacaa attcagtgaa 780

ctggccacca agtgcattat taagatcgtg gagtttgcta aacgtctgcc tggtttcact 840

ggcttgacca tcgcagacca aattaccctg ctgaaggccg cctgcctgga catcctgatt 900

cttagaattt gcaccaggta taccccagaa caagacacca tgactttctc agacggcctt 960

accctaaatc gaactcagat gcacaatgct ggatttggtc ctctgactga ccttgtgttc 1020

acctttgcca accagctcct gcctttggaa atggatgaca cagaaacagg ccttctcagt 1080

gccatctgct taatctgtgg agaccgccag gaccttgagg aaccgacaaa agtagataag 1140

ctacaagaac cattgctgga agcactaaaa atttatatca gaaaaagacg acccagcaag 1200

cctcacatgt ttccaaagat cttaatgaaa atcacagatc tccgtagcat cagtgctaaa 1260

ggtgcagagc gtgtaattac cttgaaaatg gaaattcctg gatcaatgcc acctctcatt 1320

caagaaatgc tggagaattc tgaaggacat gaacccttga ccccaagttc aagtgggaac 1380

acagcagagc acagtcctag catctcaccc agctcagtgg aaaacagtgg ggtcagtcag 1440

tcaccactcg tgcaataaga cattttctag ctacttcaaa cattccccag taccttcagt 1500

tccaggattt aaaatgcaag aaaaaacatt tttactgctg cttagttttt ggactgaaaa 1560

gatattaaaa ctcaagaagg accaagaagt tttcatatgt atcaatatat atactcctca 1620

ctgtgtaact tacctagaaa tacaaacttt tccaatttta aaaaatcagc catttcatgc 1680

aaccagaaac tagttaaaag cttctatttt cctctttgaa cactcaagat gcatggcaaa 1740

gacccagtca aaatgattta cccctggtta agtttctgaa gactttgtac atacagaagt 1800

atggctctgt tctttctata ctgtatgttt ggtgctttcc ttttgtcttg catactcaaa 1860

ataaccatga caccaaggtt atgaaataga ctactgtaca cgtctaccta ggttcaaaaa 1920

gataactgtc ttgctttcat ggaatagtca agacatcaag gtaaggaaac aggactattg 1980

acaggactat tgtacagtat gacaagataa ggctgaagat attctacttt agttagtatg 2040

gaagcttgtc tttgctcttt ctgatgctct caaactgcat cttttatttc atgttgccca 2100

gtaaaagtat acaaattccc tgcactagca gaagagaatt ctgtatcagt gtaactgcca 2160

gttcagttaa tcaaatgtca tttgttcaat tgttaatgtc actttaaatt aaaagtggtt 2220

tattacttgt ttaatgacat aactacacag ttagttaaaa aaaatttttt tacagtaatg 2280

atagcctcca aggcagaaac acttttcagt gttaagtttt tgtttacttg ttcacaagcc 2340

attagggaaa tttcatggga taattagcag gctggtctac cactggacca tgtaactcta 2400

gtgtccttcc tgattcatgc ctgatattgg gatttttttc cagcccttct tgatgccaag 2460

ggctaattat attacatccc aaagaaacag gcatagaatc tgcctccttt gaccttgttc 2520

aatcactatg aagcagagtg aaagctgtgg tagagtggtt aacagataca agtgtcagtt 2580

tcttagttct catttaagca ctactggaat tttttttttt gatatattag caagtctgtg 2640

atgtactttc actggctctg tttgtacatt gagattgttt gtttaacaat gctttctatg 2700

ttcatatact gtttaccttt ttccatggac tctcctggca aagaataaaa tatatttatt 2760

tt 2762

<210> 63
<211> 5487
<212> DNA
<213> Homo sapiens

<220>
<221> misc_feature
<223> TIMP3 >gi|21536431|ref|NM_000362.3| Homo sapiens tissue inhibitor of
metalloproteinase 3 (Sorsby fundus dystrophy, pseudoinflammatory)
(TIMP3), mRNA

<400> 63

tctgtcgact tgccccagag ctgatccttg tctttgtcca cttctcagcg aggatggcac 60
ttcagggagc ccttccctta ctatcgcaga gagagcaggc cctccccagt catgtccaac 120
ccagaactct gttttgtttt cttcatagcc ctagcatcac agaaaatcac cctgtgcatt 180
catggatgtc cacgggggca agggctttgt gttgcttaac ccagcatcct gaaccgtgtt 240
tgttgaatga atacagaacc ccgtttgctc tgggagagca cagaaaacag tcttctatca 300
tatatcatag ccagctgcaa acagcagatg gcttcccata tcccagagag taagaaccag 360
agagagagag aaagagagag agtttgggtc tttctcctct gtgcctgctc tctccagaga 420
aactggaggg gtagcagtta gcattccccc gctggttcca ccaagcacag tcaaggtctc 480
taggacatgg ccacccctca cctgtggaag cggtcctgct ggggtgggtg ggtgttagtt 540
ggttctggtt tgggtcagag acacccagtg gcccaggtgg gcgtggggcc agggcgcaga 600
cgagaagggg cacgagggct ccgctccgag gacccagcgg caagcaccgg tcccgggcgc 660
gccccagccc acccactcgc gtgcccacgg cggcattatt ccctataagg atctgaacga 720
tccgggggcg gccccgcccc gttacccctt gcccccggcc ccgccccctt tttggagggc 780
cgatgaggta atgcggctct gccattggtc tgagggggcg ggccccaaca gcccgaggcg 840
gggtccccgg gggcccagcg ctatatcact cggccgccca ggcagcggcg cagagcgggc 900
agcaggcagg cggcgggcgc tcagacggct tctcctcctc ctcttgctcc tccagctcct 960
gctccttcgc cgggaggccg cccgccgagt cctgcgccag cgccgaggca gcctcgctgc 1020
gccccatccc gtcccgccgg gcactcggag ggcagcgcgc cggaggccaa ggttgccccg 1080
cacggcccgg cgggcgagcg agctcgggct gcagcagccc cgccggcggc gcgcacggca 1140
actttggaga ggcgagcagc agccccggca gcggcggcag cagcggcaat gaccccttgg 1200
ctcgggctca tcgtgctcct gggcagctgg agcctggggg actggggcgc cgaggcgtgc 1260
acatgctcgc ccagccaccc ccaggacgcc ttctgcaact ccgacatcgt gatccgggcc 1320
aaggtggtgg ggaagaagct ggtaaaggag gggcccttcg gcacgctggt ctacaccatc 1380
aagcagatga agatgtaccg aggcttcacc aagatgcccc atgtgcagta catccacacg 1440
gaagcttccg agagtctctg tggccttaag ctggaggtca acaagtacca gtacctgctg 1500
acaggtcgcg tctatgatgg caagatgtac acggggctgt gcaacttcgt ggagaggtgg 1560
gaccagctca ccctctccca gcgcaagggg ctgaactatc ggtatcacct gggttgtaac 1620
tgcaagatca agtcctgcta ctacctgcct tgctttgtga cttccaagaa cgagtgtctc 1680
tggaccgaca tgctctccaa tttcggttac cctggctacc agtccaaaca ctacgcctgc 1740
atccggcaga agggcggcta ctgcagctgg taccgaggat gggccccccc ggataaaagc 1800
atcatcaatg ccacagaccc ctgagcgcca gaccctgccc cacctcactt ccctcccttc 1860
ccgctgagct tcccttggac actaactctt cccagatgat gacaatgaaa ttagtgcctg 1920
ttttcttgca aatttagcac ttggaacatt taaagaaagg tctatgctgt catatggggt 1980
ttattgggaa ctatcctcct ggccccaccc tgccccttct tttggttttt gacatcattc 2040

atttccacct gggaatttct ggtgccatgc cagaaagaat gaggaacctg tattcctctt 2100
cttcgtgata atataatctc tattttttta ggaaaacaaa aatgaaaaac tactccattt 2160
gaggattgta attcccaccc ctcttgcttc ttccccacct caccatctcc cagaccctct 2220
tccctttgcc cttctcctcc aatacataaa ggacacagac aaggaacttg ctgaaaggcc 2280
aaccatttca ggatcagtca aaggcagcaa gcagatagac tcaaggtgtg tgaaagatgt 2340
tatacaccag gagctgccac tgcatgtccc aaccagactg tgtctgtctg tgtctgcatg 2400
taagagtgag ggagggaagg aaggaactac aagagagtcg gagatgatgc agcacacaca 2460
caattcccca gcccagtgat gcttgtgttg accagatgtt cctgagtctg gagcaagcac 2520
ccaggccaga ataacagagc tttcttagtt ggtgaagact taaacatctg cctgaggtca 2580
ggaggcaatt tgcctgcctt gtacaaaagc tcaggtgaaa gactgagatg aatgtctttc 2640
ctctccctgc ctcccaccag acttcctcct ggaaaacgct ttggtagatt tggccaggag 2700
ctttctttta tgtaaattgg ataaatacac acaccataca ctatccacag atatagccaa 2760
gtagatttgg gtagaggata ctatttccag aatagtgttt agctcaccta gggggatatg 2820
tttgtataca catttgcata tacccacatg gggacataag ctaatttttt tacaggacac 2880
agaattctgt tcaatgctgt taaatatgcc aatagtttaa tctcttctat tttgttgtcg 2940
ttgcttgttt gaagaaaatc atgacattcc aagttgacat ttttttttca ttttaattaa 3000
aatttgaaat tctgaacacc gtcagcaccc tctcttccct atcatgggtc atctgacccc 3060
tgtccgtctc cttgtccctg cttcatgttt gggggccttt ctttaactgc cttcctggct 3120
tagctcagat ggcagatgag agtgtagtca agggcctggg cacaggaggg agagctgcag 3180
agtgtcctgc ctgccttggc tggagggaca cctctcctgg gtgtggagac agcttggttc 3240
cctttcccta gctccctggt gggtgaatgc cacctcctga gatcctcacc tcttggaatt 3300
aaaattgttg gtcactgggg aaagcctgag tttgcaacca gttgtagggt ttctgttgtg 3360
tttttttttt tttttttgaa ataaaactat aatataaatt ctcctattaa ataaaattat 3420
tttaagtttt agtgtcaaaa gtgagatgct gagagtaggt gataatgtat attttacaga 3480
gtgggggttg gcaggatggt gacattgaac atgattgctc tctgtctctt ttttcagctt 3540
atgggtattt atcttctatt agtatttgta tcttcagttc attccacttt aggaaacaga 3600
gctgccaatt gaaacagaag aagaaaaaaa aaaaaagcag cagacaacac actgtagagt 3660
cttgcacaca cacaagtgcc caggcaaggt gcttggcaga accgcagagt gggaagagag 3720
taccggcatc gggtttcctt gggatcaatt tcattaccgt gtacctttcc cattgtggtc 3780
atgccatttg gcagggggag aatgggaggc ttggccttct ttgtgaggca gtgtgagcag 3840
aagctgatgc cagcatgtca ctggttttga agggatgagc ccagacttga tgttttggga 3900
ttgtccttat tttaacctca aggtctcgca tggtggggcc cctgaccaac ctacacaagt 3960
tccctcccac aagtggacat cagtgtcttc tctgtgaggc atctggccat tcgcactccc 4020
tggtgtggtc agcctctctc acacaaggag gaacttgggt gaaggctgag tgtgaggcac 4080

ctgaagtttc cctgcggagt cgataaatta gcagaaccac atccccatct gttaggcctt 4140

ggtgaggagg ccctgggcaa agaagggtct ttcgcaaagc gatgtcagag ggcggttttg 4200

agctttctat aagctatagc tttgtttatt tcacccgttc acttactgta taatttaaaa 4260

tcatttatgt agctgagaca cttctgtatt tcaatcatat catgaacatt ttattttgct 4320

aaatcttgtg tcatgtgtag gctgtaatat gtgtacattg tgtttaagag aaaaatgaaa 4380

cccacatgcc gccattttcc tgaatcaaat tctgcagtgg aatggagagg aaaatacttc 4440

taggcaagca gctagactgg tgaattgggg gaaatagaag gaactagtaa ctgagactcc 4500

tccagcctcc tccctattgg aatcccaatg gctcctggag taggaaaaaa gtttaaacta 4560

cattcatgtt cttgttctgt gtcactcggc cctgggtagt ctaccattta cttcacccca 4620

agtcctgctg cccatccagt tgggaagcca tgattttcct aagaatccag ggccatggga 4680

gatacaattc caagttctcg cttcctcctt tgggcatctc ttctgcctcc caatcaagga 4740

agctccatgc tcaggctctc agctctcggg ccagtgctct gctctgtcca gggtaggtaa 4800

tactgggaga ctcctgtctt ttaccctccc ctcgttccag acctgcctca tggtggcaac 4860

atggttcttg aacaattaaa gaaacaaatg actttttgga atagccctgt ctagggcaaa 4920

ctgtggcccc caggagacac tacccttcca tgccccagac ctctgtcttg catgtgacaa 4980

ttgacaatct ggactacccc aagatggcac ccaagtgttt ggcttctggc tacctaaggt 5040

taacatgtca ctagagtatt tttatgagag acaaacatta taaaaatctg atggcaaaag 5100

caaaacaaaa tggaaagtag gggaggtgga tgtgacaaca acttccaaat tggctctttg 5160

gaggcgagag gaaggggaga acttggagaa tagtttttgc tttgggggta gaggcttctt 5220

agattctccc agcatccgcc tttcccttta gccagtctgc tgtcctgaaa cccagaagtg 5280

atggagagaa accaacaaga gatctcgaac cctgtctaga aggaatgtat ttgttgctaa 5340

atttcgtagc actgtttaca gttttcctcc atgttattta tgaattttat attccgtgaa 5400

tgtatattgt cttgtaatgt tgcataatgt tcacttttta tagtgtgtcc tttattctaa 5460

acagtaaagt ggttttattt ctatcac 5487

<210> 64
<211> 866
<212> DNA
<213> Homo sapiens

<220>
<221> misc_feature
<223> APC >gi|551463|gb|U02509.1|HSU02509 Human adenomatous polyposis coli
(APC) gene, promoter sequence

<220>
<221> misc_feature
<222> (315)..(315)
<223> n is a, c, g, or t

<400> 64
acttatatat ctgacagttg atttgtcctc acctctaaat tggaatttaa gcatcacctg 60

gttcgattta atgcaatgta gaatttgcat taaaatacta cattaaagcc tcagatttgt 120

agtagctaac agcacttcta tgtatgtgtc agggactgct ctaaatactt catatatatt 180

aactcctcta ttctgtactt ctgttcccgt tttatacagc aggaaattga aacactgaga 240

ggttaagtaa ctaaagttac agagctagag tgacaggagt aaagcttcaa ctcaggcaac 300

ccagacgtcc agagntctga tctccactac taagctgcta gcatagcttt tctggtaact 360

atttttaatt caatataatt cgaatgatct atctaacaag tcatcactct gacaactcag 420

tgacttgtaa tgtaaaatta ttcattgtaa ttcacttaat attattgttt ctctgtgctg 480

caaaaatcat agcaatcgag atgtaattta ttactctccc tcccacctcc ggcatcttgt 540

gctaatcctt ctgccctgcg gacctccccc gactctttac tatgcgtgtc aactgccatc 600

aacttccttg cttgctgggg actggggccg tgagggcata cccccgaggg gtacggggct 660

agggctaggc aggctgtgcg gttgggcggg gccctgtgcc ccactgcgga gtgcgggtcg 720

ggaagcggag agagaagcag ctgtgtaatc cgctggatgc ggaccagggc gctccccatt 780

cccgtcggga gcccgccgat tggctgggtg tgggcgcacg tgaccgacat gtggctgtat 840

tggtgcagcc cgccagggtg tcactg 866

<210> 65
<211> 10386
<212> DNA
<213> Homo sapiens

<220>
<221> misc_feature
<223> >gi|21626462|ref|NM_000038.2| Homo sapiens adenomatosis polyposis
coli (APC), mRNA

<220>
<221> misc_feature
<222> (9521)..(9521)
<223> n is a, c, g, or t

<400> 65
attgaggact cggaaatgag gtccaagggt agccaaggat ggctgcagct tcatatgatc 60

agttgttaaa gcaagttgag gcactgaaga tggagaactc aaatcttcga caagagctag 120

aagataattc caatcatctt acaaaactgg aaactgaggc atctaatatg aaggaagtac 180

ttaaacaact acaaggaagt attgaagatg aagctatggc ttcttctgga cagattgatt 240

tattagagcg tcttaaagag cttaacttag atagcagtaa tttccctgga gtaaaactgc 300

ggtcaaaaat gtccctccgt tcttatggaa gccgggaagg atctgtatca agccgttctg 360

gagagtgcag tcctgttcct atgggttcat ttccaagaag agggtttgta aatggaagca 420

gagaaagtac tggatattta gaagaacttg agaaagagag gtcattgctt cttgctgatc 480

ttgacaaaga agaaaaggaa aaagactggt attacgctca acttcagaat ctcactaaaa 540

gaatagatag tcttccttta actgaaaatt tttccttaca aacagatatg accagaaggc 600

aattggaata tgaagcaagg caaatcagag ttgcgatgga agaacaacta ggtacctgcc 660

aggatatgga aaaacgagca cagcgaagaa tagccagaat tcagcaaatc gaaaaggaca 720
tacttcgtat acgacagctt ttacagtccc aagcaacaga agcagagagg tcatctcaga 780
acaagcatga aaccggctca catgatgctg agcggcagaa tgaaggtcaa ggagtgggag 840
aaatcaacat ggcaacttct ggtaatggtc agggttcaac tacacgaatg gaccatgaaa 900
cagccagtgt tttgagttct agtagcacac actctgcacc tcgaaggctg acaagtcatc 960
tgggaaccaa ggtggaaatg gtgtattcat tgttgtcaat gcttggtact catgataagg 1020
atgatatgtc gcgaactttg ctagctatgt ctagctccca agacagctgt atatccatgc 1080
gacagtctgg atgtcttcct ctcctcatcc agcttttaca tggcaatgac aaagactctg 1140
tattgttggg aaattcccgg ggcagtaaag aggctcgggc cagggccagt gcagcactcc 1200
acaacatcat tcactcacag cctgatgaca agagaggcag gcgtgaaatc cgagtccttc 1260
atcttttgga acagatacgc gcttactgtg aaacctgttg ggagtggcag gaagctcatg 1320
aaccaggcat ggaccaggac aaaaatccaa tgccagctcc tgttgaacat cagatctgtc 1380
ctgctgtgtg tgttctaatg aaactttcat ttgatgaaga gcatagacat gcaatgaatg 1440
aactaggggg actacaggcc attgcagaat tattgcaagt ggactgtgaa atgtacgggc 1500
ttactaatga ccactacagt attacactaa gacgatatgc tggaatggct ttgacaaact 1560
tgacttttgg agatgtagcc aacaaggcta cgctatgctc tatgaaaggc tgcatgagag 1620
cacttgtggc ccaactaaaa tctgaaagtg aagacttaca gcaggttatt gcaagtgttt 1680
tgaggaattt gtcttggcga gcagatgtaa atagtaaaaa gacgttgcga gaagttggaa 1740
gtgtgaaagc attgatggaa tgtgctttag aagttaaaaa ggaatcaacc ctcaaaagcg 1800
tattgagtgc cttatggaat ttgtcagcac attgcactga gaataaagct gatatatgtg 1860
ctgtagatgg tgcacttgca tttttggttg gcactcttac ttaccggagc cagacaaaca 1920
ctttagccat tattgaaagt ggaggtggga tattacggaa tgtgtccagc ttgatagcta 1980
caaatgagga ccacaggcaa atcctaagag agaacaactg tctacaaact ttattacaac 2040
acttaaaatc tcatagtttg acaatagtca gtaatgcatg tggaactttg tggaatctct 2100
cagcaagaaa tcctaaagac caggaagcat tatgggacat gggggcagtt agcatgctca 2160
agaacctcat tcattcaaag cacaaaatga ttgctatggg aagtgctgca gctttaagga 2220
atctcatggc aaataggcct gcgaagtaca aggatgccaa tattatgtct cctggctcaa 2280
gcttgccatc tcttcatgtt aggaaacaaa aagccctaga agcagaatta gatgctcagc 2340
acttatcaga aacttttgac aatatagaca atttaagtcc caaggcatct catcgtagta 2400
agcagagaca caagcaaagt ctctatggtg attatgtttt tgacaccaat cgacatgatg 2460
ataataggtc agacaatttt aatactggca acatgactgt cctttcacca tatttgaata 2520
ctacagtgtt acccagctcc tcttcatcaa gaggaagctt agatagttct cgttctgaaa 2580
aagatagaag tttggagaga gaacgcggaa ttggtctagg caactaccat ccagcaacag 2640
aaaatccagg aacttcttca aagcgaggtt tgcagatctc caccactgca gcccagattg 2700

ccaaagtcat ggaagaagtg tcagccattc atacctctca ggaagacaga agttctgggt 2760
ctaccactga attacattgt gtgacagatg agagaaatgc acttagaaga agctctgctg 2820
cccatacaca ttcaaacact tacaatttca ctaagtcgga aaattcaaat aggacatgtt 2880
ctatgcctta tgccaaatta gaatacaaga gatcttcaaa tgatagttta aatagtgtca 2940
gtagtagtga tggttatggt aaaagaggtc aaatgaaacc ctcgattgaa tcctattctg 3000
aagatgatga aagtaagttt tgcagttatg gtcaataccc agccgaccta gcccataaaa 3060
tacatagtgc aaatcatatg gatgataatg atggagaact agatacacca ataaattata 3120
gtcttaaata ttcagatgag cagttgaact ctggaaggca aagtccttca cagaatgaaa 3180
gatgggcaag acccaaacac ataatagaag atgaaataaa acaaagtgag caaagacaat 3240
caaggaatca aagtacaact tatcctgttt atactgagag cactgatgat aaacacctca 3300
agttccaacc acattttgga cagcaggaat gtgtttctcc atacaggtca cggggagcca 3360
atggttcaga aacaaatcga gtgggttcta atcatggaat taatcaaaat gtaagccagt 3420
ctttgtgtca agaagatgac tatgaagatg ataagcctac caattatagt gaacgttact 3480
ctgaagaaga acagcatgaa gaagaagaga gaccaacaaa ttatagcata aaatataatg 3540
aagagaaacg tcatgtggat cagcctattg attatagttt aaaatatgcc acagatattc 3600
cttcatcaca gaaacagtca ttttcattct caaagagttc atctggacaa agcagtaaaa 3660
ccgaacatat gtcttcaagc agtgagaata cgtccacacc ttcatctaat gccaagaggc 3720
agaatcagct ccatccaagt tctgcacaga gtagaagtgg tcagcctcaa aaggctgcca 3780
cttgcaaagt ttcttctatt aaccaagaaa caatacagac ttattgtgta gaagatactc 3840
caatatgttt ttcaagatgt agttcattat catctttgtc atcagctgaa gatgaaatag 3900
gatgtaatca gacgacacag gaagcagatt ctgctaatac cctgcaaata gcagaaataa 3960
aagaaaagat tggaactagg tcagctgaag atcctgtgag cgaagttcca gcagtgtcac 4020
agcaccctag aaccaaatcc agcagactgc agggttctag tttatcttca gaatcagcca 4080
ggcacaaagc tgttgaattt tcttcaggag cgaaatctcc ctccaaaagt ggtgctcaga 4140
cacccaaaag tccacctgaa cactatgttc aggagacccc actcatgttt agcagatgta 4200
cttctgtcag ttcacttgat agttttgaga gtcgttcgat tgccagctcc gttcagagtg 4260
aaccatgcag tggaatggta agtggcatta taagccccag tgatcttcca gatagccctg 4320
gacaaaccat gccaccaagc agaagtaaaa cacctccacc acctcctcaa acagctcaaa 4380
ccaagcgaga agtacctaaa aataaagcac ctactgctga aaagagagag agtggaccta 4440
agcaagctgc agtaaatgct gcagttcaga gggtccaggt tcttccagat gctgatactt 4500
tattacattt tgccacggaa agtactccag atggattttc ttgttcatcc agcctgagtg 4560
ctctgagcct cgatgagcca tttatacaga aagatgtgga attaagaata atgcctccag 4620
ttcaggaaaa tgacaatggg aatgaaacag aatcagagca gcctaaagaa tcaaatgaaa 4680
accaagagaa agaggcagaa aaaactattg attctgaaaa ggacctatta gatgattcag 4740

atgatgatga tattgaaata ctagaagaat gtattatttc tgccatgcca acaaagtcat 4800
cacgtaaagc aaaaaagcca gcccagactg cttcaaaatt acctccacct gtggcaagga 4860
aaccaagtca gctgcctgtg tacaaacttc taccatcaca aaacaggttg caaccccaaa 4920
agcatgttag ttttacaccg ggggatgata tgccacgggt gtattgtgtt gaagggacac 4980
ctataaactt ttccacagct acatctctaa gtgatctaac aatcgaatcc cctccaaatg 5040
agttagctgc tggagaagga gttagaggag gagcacagtc aggtgaattt gaaaaacgag 5100
ataccattcc tacagaaggc agaagtacag atgaggctca aggaggaaaa acctcatctg 5160
taaccatacc tgaattggat gacaataaag cagaggaagg tgatattctt gcagaatgca 5220
ttaattctgc tatgcccaaa gggaaaagtc acaagccttt ccgtgtgaaa aagataatgg 5280
accaggtcca gcaagcatct gcgtcgtctt ctgcacccaa caaaaatcag ttagatggta 5340
agaaaaagaa accaacttca ccagtaaaac ctataccaca aaatactgaa tataggacac 5400
gtgtaagaaa aaatgcagac tcaaaaaata atttaaatgc tgagagagtt ttctcagaca 5460
acaaagattc aaagaaacag aatttgaaaa ataattccaa ggacttcaat gataagctcc 5520
caaataatga agatagagtc agaggaagtt ttgcttttga ttcacctcat cattacacgc 5580
ctattgaagg aactccttac tgtttttcac gaaatgattc tttgagttct ctagattttg 5640
atgatgatga tgttgacctt tccagggaaa aggctgaatt aagaaaggca aaagaaaata 5700
aggaatcaga ggctaaagtt accagccaca cagaactaac ctccaaccaa caatcagcta 5760
ataagacaca agctattgca aagcagccaa taaatcgagg tcagcctaaa cccatacttc 5820
agaaacaatc cacttttccc cagtcatcca aagacatacc agacagaggg gcagcaactg 5880
atgaaaagtt acagaatttt gctattgaaa atactccagt ttgcttttct cataattcct 5940
ctctgagttc tctcagtgac attgaccaag aaaacaacaa taaagaaaat gaacctatca 6000
aagagactga gccccctgac tcacagggag aaccaagtaa acctcaagca tcaggctatg 6060
ctcctaaatc atttcatgtt gaagataccc cagtttgttt ctcaagaaac agttctctca 6120
gttctcttag tattgactct gaagatgacc tgttgcagga atgtataagc tccgcaatgc 6180
caaaaaagaa aaagccttca agactcaagg gtgataatga aaaacatagt cccagaaata 6240
tgggtggcat attaggtgaa gatctgacac ttgatttgaa agatatacag agaccagatt 6300
cagaacatgg tctatcccct gattcagaaa attttgattg gaaagctatt caggaaggtg 6360
caaattccat agtaagtagt ttacatcaag ctgctgctgc tgcatgttta tctagacaag 6420
cttcgtctga ttcagattcc atcctttccc tgaaatcagg aatctctctg ggatcaccat 6480
ttcatcttac acctgatcaa gaagaaaaac cctttacaag taataaaggc ccacgaattc 6540
taaaaccagg ggagaaaagt acattggaaa ctaaaaagat agaatctgaa agtaaaggaa 6600
tcaaaggagg aaaaaaagtt tataaaagtt tgattactgg aaaagttcga tctaattcag 6660
aaatttcagg ccaaatgaaa cagccccttc aagcaaacat gccttcaatc tctcgaggca 6720
ggacaatgat tcatattcca ggagttcgaa atagctcctc aagtacaagt cctgtttcta 6780

aaaaaggccc accccttaag actccagcct ccaaaagccc tagtgaaggt caaacagcca 6840
ccacttctcc tagaggagcc aagccatctg tgaaatcaga attaagccct gttgccaggc 6900
agacatccca aataggtggg tcaagtaaag caccttctag atcaggatct agagattcga 6960
ccccttcaag acctgcccag caaccattaa gtagacctat acagtctcct ggccgaaact 7020
caatttcccc tggtagaaat ggaataagtc ctcctaacaa attatctcaa cttccaagga 7080
catcatcccc tagtactgct tcaactaagt cctcaggttc tggaaaaatg tcatatacat 7140
ctccaggtag acagatgagc caacagaacc ttaccaaaca aacaggttta tccaagaatg 7200
ccagtagtat tccaagaagt gagtctgcct ccaaaggact aaatcagatg aataatggta 7260
atggagccaa taaaaaggta gaactttcta gaatgtcttc aactaaatca agtggaagtg 7320
aatctgatag atcagaaaga cctgtattag tacgccagtc aactttcatc aaagaagctc 7380
caagcccaac cttaagaaga aaattggagg aatctgcttc atttgaatct ctttctccat 7440
catctagacc agcttctccc actaggtccc aggcacaaac tccagtttta agtccttccc 7500
ttcctgatat gtctctatcc acacattcgt ctgttcaggc tggtggatgg cgaaaactcc 7560
cacctaatct cagtcccact atagagtata atgatggaag accagcaaag cgccatgata 7620
ttgcacggtc tcattctgaa agtccttcta gacttccaat caataggtca ggaacctgga 7680
aacgtgagca cagcaaacat tcatcatccc ttcctcgagt aagcacttgg agaagaactg 7740
gaagttcatc ttcaattctt tctgcttcat cagaatccag tgaaaaagca aaaagtgagg 7800
atgaaaaaca tgtgaactct atttcaggaa ccaaacaaag taaagaaaac caagtatccg 7860
caaaaggaac atggagaaaa ataaaagaaa atgaattttc tcccacaaat agtacttctc 7920
agaccgtttc ctcaggtgct acaaatggtg ctgaatcaaa gactctaatt tatcaaatgg 7980
cacctgctgt ttctaaaaca gaggatgttt gggtgagaat tgaggactgt cccattaaca 8040
atcctagatc tggaagatct cccacaggta atactccccc ggtgattgac agtgtttcag 8100
aaaaggcaaa tccaaacatt aaagattcaa aagataatca ggcaaaacaa aatgtgggta 8160
atggcagtgt tcccatgcgt accgtgggtt tggaaaatcg cctgaactcc tttattcagg 8220
tggatgcccc tgaccaaaaa ggaactgaga taaaaccagg acaaaataat cctgtccctg 8280
tatcagagac taatgaaagt tctatagtgg aacgtacccc attcagttct agcagctcaa 8340
gcaaacacag ttcacctagt gggactgttg ctgccagagt gactcctttt aattacaacc 8400
caagccctag gaaaagcagc gcagatagca cttcagctcg gccatctcag atcccaactc 8460
cagtgaataa caacacaaag aagcgagatt ccaaaactga cagcacagaa tccagtggaa 8520
cccaaagtcc taagcgccat tctgggtctt accttgtgac atctgtttaa aagagaggaa 8580
gaatgaaact aagaaaattc tatgttaatt acaactgcta tatagacatt ttgtttcaaa 8640
tgaaacttta aaagactgaa aaattttgta aataggtttg attcttgtta gagggttttt 8700
gttctggaag ccatatttga tagtatactt tgtcttcact ggtcttattt tgggaggcac 8760
tcttgatggt taggaaaaaa atagtaaagc caagtatgtt tgtacagtat gttttacatg 8820

tatttaaagt agcatcccat cccaacttcc tttaattatt gcttgtctta aaataatgaa 8880
cactacagat agaaaatatg atatattgct gttatcaatc atttctagat tataaactga 8940
ctaaacttac atcagggaaa aattggtatt tatgcaaaaa aaaatgtttt tgtccttgtg 9000
agtccatcta acatcataat taatcatgtg gctgtgaaat tcacagtaat atggttcccg 9060
atgaacaagc tttacccagc ctgtttgctt tactgcatga atgaaactga tggttcaatt 9120
tcagaagtaa tgattaacag ttatgtggtc acatgatgtg catagagata gctacagtgt 9180
aataatttac actattttgt gctccaaaca aaacaaaaat ctgtgtaact gtaaaacatt 9240
gaatgaaact attttacctg aactagattt tatctgaaag taggtagaat ttttgctatg 9300
ctgtaatttg ttgtatattc tggtatttga ggtgagatgg ctgctctttt attaatgaga 9360
catgaattgt gtctcaacag aaactaaatg aacatttcag aataaattat tgctgtatgt 9420
aaactgttac tgaaattggt atttgtttga agggtcttgt ttcacatttg tattaataat 9480
tgtttaaaat gcctctttta aaagcttata taaatttttt ncttcagctt ctatgcatta 9540
agagtaaaat tcctcttact gtaataaaaa caattgaaga agactgttgc cacttaacca 9600
ttccatgcgt tggcacttat ctattcctga aattctttta tgtgattagc tcatcttgat 9660
ttttaacatt tttccactta aacttttttt tcttactcca ctggagctca gtaaaagtaa 9720
attcatgtaa tagcaatgca agcagcctag cacagactaa gcattgagca taataggccc 9780
acataatttc ctctttctta atattataga aattctgtac ttgaaattga ttcttagaca 9840
ttgcagtctc ttcgaggctt tacagtgtaa actgtcttgc cccttcatct tcttgttgca 9900
actgggtctg acatgaacac tttttatcac cctgtatgtt agggcaagat ctcagcagtg 9960
aagtataatc agcactttgc catgctcaga aaattcaaat cacatggaac tttagaggta 10020
gatttaatac gattaagata ttcagaagta tattttagaa tccctgcctg ttaaggaaac 10080
tttatttgtg gtaggtacag ttctggggta catgttaagt gtccccttat acagtggagg 10140
gaagtcttcc ttcctgaagg aaaataaact gacacttatt aactaagata atttacttaa 10200
tatatcttcc ctgatttgtt ttaaaagatc agagggtgac tgatgataca tgcatacata 10260
tttgttgaat aaatgaaaat ttatttttag tgataagatt catacactct gtatttgggg 10320
agagaaaacc tttttaagca tggtggggca ctcagatagg agtgaataca cctacctggt 10380
ggtcat 10386

<210> 66
<211> 7273
<212> DNA
<213> Homo sapiens

<220>
<221> misc_feature
<223> PTGS2 >gi|3282785|gb|AF044206.1| Homo sapiens cyclooxygenase (COX-2)
gene, promoter and exon 1

<400> 66
ggtacccagg ctggagtgca ctggtgtgat catagctcac taacctcgaa ctcctgggct 60
taggcaatcc tcttgccttg gcctcccaaa gtgccaggat tacaggcatg agccaccaca 120
gtggagctct caattctgat actaataatt tgtgtcttct ctttttttcc ttagcctgac 180
tagagtaatt aactttatgt cttttaaaag aaccaccttt ttggttttac ccattttctt 240
ttttgatttt ctgtttttga tttgattgat atctactcta attttttatt atttcttttc 300
ctctgcttac tttgaattta attacttttc ttttttgtag tctcctaaaa tagaagctta 360
tattattgat tttagatctt tcttcttttc tattacagca ctcaatgcta taaatttccc 420
tctaagcatt gctttcactg catcctacaa tatttcaact ctattgttat ttagctcaaa 480
agaggttctt aatttctatt gggatttctc tttgacccat gtgttattca gaagtgttcc 540
gtgtgatctc caaatatttg ggagtttttc agctatcttt ctattaatca tttcttgttt 600
aattctattg tggcctgaga gcatatattg tatgatttat attcttgtaa atgtgttaag 660
gtgtgtctta tggtgcagaa tgcggtttat cttgctatat gttccttaga gaataatgta 720
tgttctgctg ttattggata aagtagtcta tagatgtcag ttacatctcg ttgattaatg 780
gtgctgttga gttcagctat gtcctaaatg attttctgtc tgctgtatct gtctatttct 840
gacacaaggc tgttgaagtc tccaaccata ataatgaatt aatctatttt tctttgcagt 900
tttatcaatt ttgtcttata tatattgatg ctccattgtt tggcacatac acattaagaa 960
ttgttatgtc ttcttggaga atttaccttt ccataacatg taacatttcc ctttattcct 1020
gataattttt cttgctcaaa agtttgccct gttggaaatt accagaacta ctctggcttt 1080
atttgattag tgttagcatg ctctctcttt ctctattctt acacttttaa tgtatacttg 1140
actttgtatt taaagtgggg ttcttataga aaacatatac ttggtagggt gggaagtaaa 1200
ataaaaagaa atacttgggt attggtttga tccactctaa caatctctat gttttaattg 1260
atgtatttag accattgata cttatttttt tatcctcatc cctgtgatta cccagagagc 1320
tgcttaaatt gattattgat atagacaaat taataattaa tatctaccgt ttgttactgt 1380
tttctatttt tcattgccct tactttctgc tcctattttt tgctcctttt tctgttaatt 1440
taggttttga gttattttat atcattctat tttctctccc ttctcagcat atgaattatc 1500
tttctttttg acttttttag tggctgccct gaaggttgca atgtacattt acaaccagtc 1560
ccaatctcct ttcaaaaaac acaatactgt ttcatggcta gtgcaagtac ctaataataa 1620
gaagtcactc ctaatttctt tctctcattc tttgtatctt tactgttatt catttcactt 1680
gtacataagc tgtaatcttt caatacatta ttgctattat tatttcaaaa catgttatct 1740
attatatcta tttaaaataa gaaaaatagg ccaggtgcag tggcttactc atgtaatccc 1800
agcactttgg gagaccgatg gattgctaga gctcaggaat tcgagaccag cctgggcaac 1860
atagtgaaac cctgtctcta ctaaaaatac aaaaaaaaaa attgctgggc atggtggcat 1920
gggcctgtgg tcacagctac tcgggaggct gaggtgagag gattgcttga gcctgggagg 1980
cagaggttgc agtgaaccaa aatcaagcta ctgcactcca gcctaagtga cagagtgaga 2040

ccctgtctca aaaaaaaaat gaaagaatta tttttattta tcttcactta tttcttctct 2100
aatgctcttt gtttctttag tatgtagatc caagtttcta acctgtatca tttttcttat 2160
ctcaataact tcttttaaca tttctcacaa agcagatcta ctggccacag aatgcctcaa 2220
ttttcatttg tctgagaaaa ccttatttct ccttcacttt tgaaagataa ttttgtaggg 2280
tacagaattc taggttgtag gttttttccc ctcaaagtga aatatttcat tccactcttt 2340
tcttctttgt atggtatctg agaagaagtc agatgtaatt cttatcatta ttacttaaaa 2400
gattgcttct gttcctttct ctcttctcct tcccttcttt ccttctctgt atattacacc 2460
ttttatagtt gccccatatt tcttagatat tatgttttgg ttttcttctg tgtttttttc 2520
tttgattctc agttttagaa gtctctattt atatatctgc aatcgcaggg attctttcct 2580
ctgccatgtc cagtctacta ataagccctt acagacattg ttgacttctg ttccagtgtt 2640
tttgatctct agcatttctc tgattatttc ttggaattgc catctgtcta cttacattac 2700
caacctattc ttgtgtgttg tcttatcata gtaattgcag ttgttttaat ttcataggta 2760
ttgtaatttc aacatctcta ccatatttga cattgattct gatgcttgct ctgtcttatc 2820
aagctatgtt tttgtctttt agtgtgactt ctaatttttt gttgaaagcc aggcatgatg 2880
tactgagtga aagaaactca atacattgta atgtgacgat aagagttcag gggaagtgaa 2940
gcattctata gtcctatagc aggtctcggc cttttagtga gcctgtgcct atgaacggtg 3000
actttcaaca agtgcttttc attccactct tttcctgtcc ttaagtggga caagatcact 3060
gggggggggc tagaattggg tatttccctt ctccaatgta gaagctaaag agagggctgg 3120
agttgggtat ttttcttccc ctgtatggaa agctagaggc agttaaattt ggatattttc 3180
cttcttctaa ttcagttagg ctgcgacaaa aatcccgaca gtttaggctc taatattata 3240
aaataatttc tcttgagtat aggccttatt aagaacacta tactctgatg gagctgaggg 3300
ggagttttct ctgatattca ctgcgagaac ctcgtagagc tccaggaagc aaaactcaca 3360
aaagtgtggg agtcttccag aatttttcct ttgcagactt atctgcactg aacctccaga 3420
aattcatcaa ttacagttca ggttttccta cccaggtact ggttttcatg gaggtttctg 3480
cctgtgcatt tctgctccag taagttgttc ttcttgtatg gtctgtcttt caaatttttt 3540
aagtagggtt atgacctgtc gcctcacttc tctgacagtt ctgagagtgt tgatttttca 3600
gtttgcttag atttttactt gtttttagga tgaagtgaca atttccaagc tcctccctga 3660
catgccagat cagaaactga aagtcctaag cctcatattc tgtgcgtggg tatgttcaca 3720
tcctgcctgc tccagtgccc ccacctcaca ctctctttcc cttccttgtc cccttgtgag 3780
atttctaggt ccaatacaaa gactgtgttc aactcattca actacttggc tcatctgagt 3840
attataatga acaatcacaa aaaaaaatga agtaaaagaa aaatccatca aagaattgag 3900
atatttgaga aaaagaaagg agatcagtgt tttataaaac ttagaaatag attttttaag 3960
tgtttcttca ttgacttatg tgaaaggact tttcttaatt taacaaatta tgtgctttcg 4020
tttatagcct caaaacttct tgtgtagcta agaatgggta aataatcagg ctttactaaa 4080

ggactaacgt aaagatcttc tgtaagtaac atttctgcta ctcaaggaag agataaactt 4140
catggcataa ccttgccaaa gtatactaag aataaccctg acacaaagct cttttttcag 4200
ccaacatgcc atgaaagaaa gaagacaagg ggtgatctcc actctctaag tgaaccacta 4260
aacccaccaa agaagaaacg agggaaatag aaagaggacc cttgcctgag ataatggatc 4320
tgtatgtatg agtagtagaa ccctgctcaa agtacaagga agggaaaaaa aagttagttt 4380
atttggaatt ttggacatta agagtcttta ttgttcattt tcttttaact cacatgaatg 4440
gcttatcact tcaattaata aatatttcat ttcttttcaa tctatattca tgaaacaaat 4500
ctgaaatgaa cagtgcaaca tgtgaatgtt tagaacatta taaaattaaa cacaaaatct 4560
gtctggcaat cttcctagca tcttaggaaa aaagttgaca aaatttcaag cagcagaagg 4620
gggcagtaaa actcaacaga aagctctgga agatttttaa gattcttcct tattttcttt 4680
tcatgtagag tatttcccaa caaatttcag acgctaatag aaattttgta caacagatcc 4740
atatatttgc ctaaaataga cacagaaaca ttgatatatg caaacatgag agctataagt 4800
tttacatgat caaaaccttt tttttatggt acacaatagt cacagtactt ttccatataa 4860
aacaggttta gtggtcttaa tttagtttgg cacatttaat acactcccat gaccagcatc 4920
ccaaatgtac ctatccgttt tattttattg tctcagaatt gtcagttatt taataaatta 4980
tgtaactttt ttccttatgc tcagatttgc acttctttct aaaactctgc ccatccttaa 5040
agtcccagat tctccttgaa cttttttttt tgactttcca agtacatgga actcttcact 5100
ctatcctgct atataagtga cagaatttcc actatgggat agatggagtt caattccttt 5160
gagtttaaaa taatctaaat ataattattc cttatgccct gtttttccct cacttttgta 5220
tccaaatctc ttttcagaca acagaacaat taatgtctga taaggaagac aatgatgatg 5280
atcacttcaa aataagcttg aattcaggat tgtaatgtaa aattttagta ctctctcaca 5340
gtatggattc taacatggct tctaacccaa actaacatta gtagctctaa ctataaactt 5400
caaatttcag tagatgcaac ctactccttt aaaatgaaac agaagattga aattattaaa 5460
ttatcaaaaa gaaaatgatc cacgctctta gttgaaattt catgtaagat tccatgcaat 5520
aaataggagt gccataaatg gaatgatgaa atatgactag aggaggagaa aggcttccta 5580
gatgagatgg aattttagtc atccgtgtct catgaagaat cagatgtgta cactaagcaa 5640
aacagttaaa aaaaaaacct ccaagtgagt ctcttattta tttttttctt ataagacttc 5700
tacaaattga ggtacctggt gtagttttat ttcaggtttt atgctgtcat tttcctgtaa 5760
tgctaaggac ttaggacata actgaatttt ctattttcca cttcttttct ggtgtgtgtg 5820
tatatatata tgtatatata cacacacaca tatacatata tatattttta gtatctcacc 5880
ctcacatgct cctccctgag cactacccat gatagatgtt aaacaaaagc aaagatgaaa 5940
ttccaactgt taaaatctcc cttccatcta attaattcct catccaacta tgttccaaaa 6000
cgagaataga aaattagccc caataagccc aggcaactga aaagtaaatg ctatgttgta 6060
ctttgatcca tggtcacaac tcataatctt ggaaaagtgg acagaaaaga caaaagagtg 6120

aactttaaaa ctcgaattta ttttaccagt atctcctatg aagggctagt aaccaaaata 6180

atccacgcat cagggagaga aatgccttaa ggcatacgtt ttggacattt agcgtccctg 6240

caaattctgg ccatcgccgc ttcctttgtc catcagaagg caggaaactt tatattggtg 6300

acccgtggag ctcacattaa ctatttacag ggtaactgct taggaccagt attatgagga 6360

gaatttacct ttcccgcctc tctttccaag aaacaaggag ggggtgaagg tacggagaac 6420

agtatttctt ctgttgaaag caacttagct acaaagataa attacagcta tgtacactga 6480

aggtagctat ttcattccac aaaataagag ttttttaaaa agctatgtat gtatgtcctg 6540

catatagagc agatatacag cctattaagc gtcgtcacta aaacataaaa catgtcagcc 6600

tttcttaacc ttactcgccc cagtctgtcc cgacgtgact tcctcgaccc tctaaagacg 6660

tacagaccag acacggcggc ggcggcggga gaggggattc cctgcgcccc cggacctcag 6720

ggccgctcag attcctggag aggaagccaa gtgtccttct gccctccccc ggtatcccat 6780

ccaaggcgat cagtccagaa ctggctctcg gaagcgctcg ggcaaagact gcgaagaaga 6840

aaagacatct ggcggaaacc tgtgcgcctg gggcggtgga actcggggag gagagggagg 6900

gatcagacag gagagtgggg actaccccct ctgctcccaa attggggcag cttcctgggt 6960

ttccgatttt ctcatttccg tgggtaaaaa accctgcccc caccgggctt acgcaatttt 7020

tttaagggga gaggagggaa aaatttgtgg ggggtacgaa aaggcggaaa gaaacagtca 7080

tttcgtcaca tgggcttggt tttcagtctt ataaaaagga aggttctctc ggttagcgac 7140

caattgtcat acgacttgca gtgagcgtca ggagcacgtc caggaactcc tcagcagcgc 7200

ctccttcagc tccacagcca gacgccctca gacagcaaag cctacccccc gcgccgcgcc 7260

ctgcccgaag ctt 7273

<210> 67
<211> 4465
<212> DNA
<213> Homo sapiens

<220>
<221> misc_feature
<223> >gi|4506264|ref|NM_000963.1| Homo sapiens
prostaglandin-endoperoxide synthase 2 (prostaglandin G/H synthase
and cyclooxygenase) (PTGS2), mRNA

<400> 67
caattgtcat acgacttgca gtgagcgtca ggagcacgtc caggaactcc tcagcagcgc 60

ctccttcagc tccacagcca gacgccctca gacagcaaag cctacccccg cgccgcgccc 120

tgcccgccgc tcggatgctc gcccgcgccc tgctgctgtg cgcggtcctg gcgctcagcc 180

atacagcaaa tccttgctgt tcccacccat gtcaaaaccg aggtgtatgt atgagtgtgg 240

gatttgacca gtataagtgc gattgtaccc ggacaggatt ctatggagaa aactgctcaa 300

caccggaatt tttgacaaga ataaaattat ttctgaaacc cactccaaac acagtgcact 360

acatacttac ccacttcaag ggattttgga acgttgtgaa taacattccc ttccttcgaa 420

atgcaattat gagttatgtc ttgacatcca gatcacattt gattgacagt ccaccaactt 480
acaatgctga ctatggctac aaaagctggg aagccttctc taacctctcc tattatacta 540
gagcccttcc tcctgtgcct gatgattgcc cgactccctt gggtgtcaaa ggtaaaaagc 600
agcttcctga ttcaaatgag attgtggaaa aattgcttct aagaagaaag ttcatccctg 660
atccccaggg ctcaaacatg atgtttgcat tctttgccca gcacttcacg catcagtttt 720
tcaagacaga tcataagcga gggccagctt tcaccaacgg gctgggccat ggggtggact 780
taaatcatat ttacggtgaa actctggcta gacagcgtaa actgcgcctt ttcaaggatg 840
gaaaaatgaa atatcagata attgatggag agatgtatcc tcccacagtc aaagatactc 900
aggcagagat gatctaccct cctcaagtcc ctgagcatct acggtttgct gtggggcagg 960
aggtctttgg tctggtgcct ggtctgatga tgtatgccac aatctggctg cgggaacaca 1020
acagagtatg cgatgtgctt aaacaggagc atcctgaatg gggtgatgag cagttgttcc 1080
agacaagcag gctaatactg ataggagaga ctattaagat tgtgattgaa gattatgtgc 1140
aacacttgag tggctatcac ttcaaactga aatttgaccc agaactactt ttcaacaaac 1200
aattccagta ccaaaatcgt attgctgctg aatttaacac cctctatcac tggcatcccc 1260
ttctgcctga cacctttcaa attcatgacc agaaatacaa ctatcaacag tttatctaca 1320
acaactctat attgctggaa catggaatta cccagtttgt tgaatcattc accaggcaaa 1380
ttgctggcag ggttgctggt ggtaggaatg ttccacccgc agtacagaaa gtatcacagg 1440
cttccattga ccagagcagg cagatgaaat accagtcttt taatgagtac cgcaaacgct 1500
ttatgctgaa gccctatgaa tcatttgaag aacttacagg agaaaaggaa atgtctgcag 1560
agttggaagc actctatggt gacatcgatg ctgtggagct gtatcctgcc cttctggtag 1620
aaaagcctcg gccagatgcc atctttggtg aaaccatggt agaagttgga gcaccattct 1680
ccttgaaagg acttatgggt aatgttatat gttctcctgc ctactggaag ccaagcactt 1740
ttggtggaga agtgggtttt caaatcatca acactgcctc aattcagtct ctcatctgca 1800
ataacgtgaa gggctgtccc tttacttcat tcagtgttcc agatccagag ctcattaaaa 1860
cagtcaccat caatgcaagt tcttcccgct ccggactaga tgatatcaat cccacagtac 1920
tactaaaaga acgttcgact gaactgtaga agtctaatga tcatatttat ttatttatat 1980
gaaccatgtc tattaattta attatttaat aatatttata ttaaactcct tatgttactt 2040
aacatcttct gtaacagaag tcagtactcc tgttgcggag aaaggagtca tacttgtgaa 2100
gacttttatg tcactactct aaagattttg ctgttgctgt taagtttgga aaacagtttt 2160
tattctgttt tataaaccag agagaaatga gttttgacgt ctttttactt gaatttcaac 2220
ttatattata agaacgaaag taaagatgtt tgaatactta aacactatca caagatggca 2280
aaatgctgaa agtttttaca ctgtcgatgt ttccaatgca tcttccatga tgcattagaa 2340
gtaactaatg tttgaaattt taaagtactt ttggttattt ttctgtcatc aaacaaaaac 2400
aggtatcagt gcattattaa atgaatattt aaattagaca ttaccagtaa tttcatgtct 2460

actttttaaa atcagcaatg aaacaataat ttgaaatttc taaattcata gggtagaatc 2520
acctgtaaaa gcttgtttga tttcttaaag ttattaaact tgtacatata ccaaaaagaa 2580
gctgtcttgg atttaaatct gtaaaatcag atgaaatttt actacaattg cttgttaaaa 2640
tattttataa gtgatgttcc tttttcacca agagtataaa cctttttagt gtgactgtta 2700
aaacttcctt ttaaatcaaa atgccaaatt tattaaggtg gtggagccac tgcagtgtta 2760
tctcaaaata agaatatttt gttgagatat tccagaattt gtttatatgg ctggtaacat 2820
gtaaaatcta tatcagcaaa agggtctacc tttaaaataa gcaataacaa agaagaaaac 2880
caaattattg ttcaaattta ggtttaaact tttgaagcaa actttttttt atccttgtgc 2940
actgcaggcc tggtactcag attttgctat gaggttaatg aagtaccaag ctgtgcttga 3000
ataacgatat gttttctcag attttctgtt gtacagttta atttagcagt ccatatcaca 3060
ttgcaaaagt agcaatgacc tcataaaata cctcttcaaa atgcttaaat tcatttcaca 3120
cattaatttt atctcagtct tgaagccaat tcagtaggtg cattggaatc aagcctggct 3180
acctgcatgc tgttcctttt cttttcttct tttagccatt ttgctaagag acacagtctt 3240
ctcatcactt cgtttctcct attttgtttt actagtttta agatcagagt tcactttctt 3300
tggactctgc ctatattttc ttacctgaac ttttgcaagt tttcaggtaa acctcagctc 3360
aggactgcta tttagctcct cttaagaaga ttaaaagaga aaaaaaaagg cccttttaaa 3420
aatagtatac acttatttta agtgaaaagc agagaatttt atttatagct aattttagct 3480
atctgtaacc aagatggatg caaagaggct agtgcctcag agagaactgt acggggtttg 3540
tgactggaaa aagttacgtt cccattctaa ttaatgccct ttcttattta aaaacaaaac 3600
caaatgatat ctaagtagtt ctcagcaata ataataatga cgataatact tcttttccac 3660
atctcattgt cactgacatt taatggtact gtatattact taatttattg aagattatta 3720
tttatgtctt attaggacac tatggttata aactgtgttt aagcctacaa tcattgattt 3780
ttttttgtta tgtcacaatc agtatatttt ctttggggtt acctctctga atattatgta 3840
aacaatccaa agaaatgatt gtattaagat ttgtgaataa atttttagaa atctgattgg 3900
catattgaga tatttaaggt tgaatgtttg tccttaggat aggcctatgt gctagcccac 3960
aaagaatatt gtctcattag cctgaatgtg ccataagact gaccttttaa aatgttttga 4020
gggatctgtg gatgcttcgt taatttgttc agccacaatt tattgagaaa atattctgtg 4080
tcaagcactg tgggttttaa tatttttaaa tcaaacgctg attacagata atagtattta 4140
tataaataat tgaaaaaaat tttcttttgg gaagagggag aaaatgaaat aaatatcatt 4200
aaagataact caggagaatc ttctttacaa ttttacgttt agaatgttta aggttaagaa 4260
agaaatagtc aatatgcttg tataaaacac tgttcactgt tttttttaaa aaaaaaactt 4320
gatttgttat taacattgat ctgctgacaa aacctgggaa tttgggttgt gtatgcgaat 4380
gtttcagtgc ctcagacaaa tgtgtattta acttatgtaa aagataagtc tggaaataaa 4440
tgtctgttta tttttgtact attta 4465

<210>  68
<211>  1336
<212>  DNA
<213>  Homo sapiens

<220>
<221>  misc_feature
<223>  14-3-3-Sigma      >gi|45238846|ref|NM_006142.3| Homo sapiens stratifin
(SFN), mRNA

<400>  68
gagagacaca gagtccggca ttggtcccag gcagcagtta gcccgccgcc cgcctgtgtg      60

tccccagagc catggagaga gccagtctga tccagaaggc caagctggca gagcaggccg     120

aacgctatga ggacatggca gccttcatga aaggcgccgt ggagaagggc gaggagctct     180

cctgcgaaga gcgaaacctg ctctcagtag cctataagaa cgtggtgggc ggccagaggg     240

ctgcctggag ggtgctgtcc agtattgagc agaaaagcaa cgaggagggc tcggaggaga     300

aggggcccga ggtgcgtgag taccgggaga aggtggagac tgagctccag ggcgtgtgcg     360

acaccgtgct gggcctgctg gacagccacc tcatcaagga ggccggggac gccgagagcc     420

gggtcttcta cctgaagatg aagggtgact actaccgcta cctggccgag gtggccaccg     480

gtgacgacaa gaagcgcatc attgactcag cccggtcagc ctaccaggag gccatggaca     540

tcagcaagaa ggagatgccg cccaccaacc ccatccgcct gggcctggcc ctgaactttt     600

ccgtcttcca ctacgagatc gccaacagcc ccgaggaggc catctctctg gccaagacca     660

ctttcgacga ggccatggct gatctgcaca ccctcagcga ggactcctac aaagacagca     720

ccctcatcat gcagctgctg cgagacaacc tgacactgtg gacggccgac aacgccgggg     780

aagagggggg cgaggctccc caggagcccc agagctgagt gttgcccgcc accgccccgc     840

cctgccccct ccagtccccc accctgccga gaggactagt atggggtggg aggccccacc     900

cttctcccct aggcgctgtt cttgctccaa agggctccgt ggagagggac tggcagagct     960

gaggccacct ggggctgggg atcccactct tcttgcagct gttgagcgca cctaaccact    1020

ggtcatgccc ccacccctgc tctccgcacc cgcttcctcc cgaccccagg accaggctac    1080

ttctcccctc ctcttgcctc cctcctgccc ctgctgcctc tgatcgtagg aattgaggag    1140

tgtcccgcct tgtggctgag aactggacag tggcaggggc tggagatggg tgtgtgtgtg    1200

tgtgtgtgtg tgtgtgtgtg tgtgcgcgcg cgccagtgca agaccgagat tgagggaaag    1260

catgtctgct gggtgtgacc atgtttcctc tcaataaagt tcccctgtga cactcaaaaa    1320

aaaaaaaaaa aaaaaa                                                    1336

<210>  69
<211>  1968
<212>  DNA
<213>  Homo sapiens

<220>

<221>  misc_feature
<223>  RASSF1A      >gi|25777678|ref|NM_007182.4| Homo sapiens Ras association
      (RalGDS/AF-6) domain family 1 (RASSF1), transcript variant A,mRNA

<400>  69
   tctcctcagc tccttcccgc cgcccagtct ggatcctggg ggaggcgctg aagtcggggc      60
   ccgccctgtg gccccgcccg gcccgcgctt gctagcgccc aaagccagcg aagcacgggc     120
   ccaaccgggc catgtcgggg gagcctgagc tcattgagct gcgggagctg gcacccgctg     180
   ggcgcgctgg gaagggccgc acccggctgg agcgtgccaa cgcgctgcgc atcgcgcggg     240
   gcaccgcgtg caaccccaca cggcagctgg tccctggccg tggccaccgc ttccagcccg     300
   cggggcccgc cacgcacacg tggtgcgacc tctgtggcga cttcatctgg ggcgtcgtgc     360
   gcaaaggcct gcagtgcgcg cattgcaagt tcacctgcca ctaccgctgc cgcgcgctcg     420
   tctgcctgga ctgttgcggg ccccgggacc tgggctggga acccgcggtg gagcgggaca     480
   cgaacgtgga cgagcctgtg gagtgggaga cacctgacct ttctcaagct gagattgagc     540
   agaagatcaa ggagtacaat gcccagatca acagcaacct cttcatgagc ttgaacaagg     600
   acggttctta cacaggcttc atcaaggttc agctgaagct ggtgcgccct gtctctgtgc     660
   cctccagcaa gaagccaccc tccttgcagg atgcccggcg gggcccagga cggggcacaa     720
   gtgtcaggcg ccgcacttcc ttttacctgc ccaaggatgc tgtcaagcac ctgcatgtgc     780
   tgtcacgcac aagggcacgt gaagtcattg aggccctgct gcgaaagttc ttggtggtgg     840
   atgacccccg caagtttgca ctctttgagc gcgctgagcg tcacggccaa gtgtacttgc     900
   ggaagctgtt ggatgatgag cagcccctgc ggctgcggct cctggcaggg cccagtgaca     960
   aggccctgag ctttgtcctg aaggaaaatg actctgggga ggtgaactgg gacgccttca    1020
   gcatgcctga actacataac ttcctacgta tcctgcagcg ggaggaggag gagcacctcc    1080
   gccagatcct gcagaagtac tcctattgcc gccagaagat ccaagaggcc ctgcacgcct    1140
   gcccccttgg gtgacctctt gtacccccag gtggaaggca gacagcaggc agcgccaagt    1200
   gcgtgccgtg tgagtgtgac agggccagtg gggcctgtgg aatgagtgtg catggaggcc    1260
   ctcctgtgct gggggaatga gcccagagaa cagcgaagta gcttgctccc tgtgtccacc    1320
   tgtgggtgta gccaggtatg gctctgcacc cctctgccct cattactggg ccttagtggg    1380
   ccagggctgc cctgagaagc tgctccaggc ctgcagcagg agtggtgcag acagaagtct    1440
   cctcaatttt tgtctcagaa gtgaaaatct tggagaccct gcaaacagaa cagggtcatg    1500
   tttgcagggg tgacggccct catctatgag gaaaggtttt ggatcttgaa tgtggtctca    1560
   ggatatcctt atcagagcta agggtgggtg ctcagaataa ggcaggcatt gaggaagagt    1620
   cttggtttct ctctacagtg ccaactcctc acacaccctg aggtcaggga gtgctggctc    1680
   acagtacagc atgtgcctta atgcttcata tgaggaggat gtccctgggc cagggtctgt    1740
   gtgaatgtgg gcactggccc aggttcatac cttatttgct aatcaaagcc agggtctctc    1800
   cctcaggtgt tttttatgaa gtgcgtgaat gtatgtaatg tgtggtggcc tcagctgaat    1860
   gcctcctgtg ggaaaggggg ttggggtgac agtcatcatc agggcctggg gcctgagaga    1920
   attggctcaa taaagatttc aagatcctca aaaaaaaaaa aaaaaaaa 1968

## Description

### BACKGROUND OF THE INVENTION

This invention relates to the interrogation of methylated genes in concert with other diagnostic methods and kits for use with these methods.

In higher order eukaryotes DNA is methylated only at cytosines located 5' to guanosine in the CpG dinucleotide. This modification has important regulatory effects on gene expression, especially when it involves CpG rich areas (CpG islands) located in gene promoter regions. Abberant methylation of normally unmethylated CpG islands is a frequent event in immortalized and transformed cells and has been associated with transcriptional inactivation of certain tumor suppressor genes or genes otherwise associated with the amelioration of certain human cancers.

Glutathione S-transferases (GSTs) are exemplary proteins in which the methylation status of the genes that express them can have important prognostic and diagnostic value for prostate cancer. The proteins catalyze intracellular detoxification reactions, including the inactivation of electrophilic carcinogens, by conjugating chemically-reactive electrophiles to glutathione (C. B. Pickett, et al., Annu. Rev. Blocbern., 58:743, 1989; B. Coles, et al., CRC Crit. Rev. Biochem. Mol. Biol., 25:47, 1990; T. H. Rushmore, et al., J. Biol. Chem. 268:11475, 1993). Human GSTs, encoded by several different genes at different loci, have been classified into four families referred to as alpha, mu, pi, and theta (B. Mannervik, et al., Biochem. J., 282:305, 1992). Decreased GSTP1 expression resulting from epigenetic changes is often related to prostate and hepatic cancers.

A commonly used system for determining the prognosis of a patient with prostate cancer is the Gleason scoring system. The Gleason scoring system is based on microscopic tumor patterns assessed by a pathologist while interpreting a biopsy specimen from a patient's prostate. Nomograms have also been developed by Kattan and others in which prognosis includes the Gleason score and a number of other factors.

Gleason scores are assessed when prostate cancer is present in a prostate biopsy. The Gleason score is based upon the degree of loss of the normal glandular tissue architecture (i.e. shape, size and differentiation of the glands) as originally described and developed by Dr. Donald Gleason. See, Gleason DF, Mellinger GT, and the Veterans Administration Cooperative Urological Research Group: Prediction of prognosis for prostatic adenocarcinoma by combined histologic grading and clinical staging. J Urol 111:58-64, 1974. The classic Gleason scoring diagram shows five basic tissue patterns that are referred to as tumor "grades". The subjective microscopic determination of this loss of normal glandular structure caused by the cancer is abstractly represented by a grade, a number ranging from 1 to 5, with 5 being the worst grade possible. The Gleason score (GS) and the Gleason sum are one and the same. However, the "Gleason grade" and the "Gleason score" (also referred to as the "Gleason sum") are different. The Gleason score is a sum of the primary grade (representing the majority of tumor) and a secondary grade (assigned to the minority of the tumor), and is a number ranging from 2 to 10. Under current practice, it is widely held that the higher the Gleason score, the more aggressive the tumor is likely to be and the worse the patient's prognosis. While useful, the correlation between Gleason score and cancer prognosis is not straight-forward. For one thing, samples with a Gleason score of 7 or greater represent a heterogeneous group of cancers and this heterogeneity can detract from predicatability. It is important to sub-stratify cancers exhibiting Gleason scores of 7 or more because the nature of the therapy provided to a patient depends upon it.

With respect to diagnostics and prognostics that do not involve biopsy samples, it is well known that Prostate Specific Antigen ("PSA") is the standard "marker" for prostate cancer. The use of the marker is helpful but not determinative in diagnostic applications and the marker is of minimal use as a prognostic. New techniques for improving the use of known markers such as PSA would also be beneficial.

The present invention fulfills these needs.

### SUMMARY OF THE INVENTION

In one aspect of the invention, a method for characterizing prostate cancer in a patient comprises determining the Gleason score of the patient and detecting epigenetic changes such as gene methylation in the patient if his Gleason score is seven or greater. The cancer is characterized as aggressive if the degree or amount of epigenetic change exceeds a predetermined value and indolent if it does not. The patient is treated consistent with the manner in which those with aggressive or indolent prostate cancers are treated.

In one aspect of the invention, methylation of one or more genes from the following group is detected: GSTP1, APC, RASSF1A, 15-LO-1, and CDH1. Preferably, the methylation status of the GSTP1 promoter is detected in blood, a blood component, urine, urethral washings, ejaculate, or tissue sample. Most preferably, the sample is a tissue sample.

In another aspect of the invention, a Gleason score is obtained for a prostate cancer patient. If the patient is assessed as having a Gleason score of 7 or higher, another biological sample is taken from the patient or the sample from which the Gleason score was adduced is further assayed. A nucleic acid sample suspected of having methylated target sequences is obtained from one or both biological samples, the sample is treated with a reagent that can prime a portion of a nucleic acid target, the nucleic acid target is primed, and the degree of methylation of the amplified target from the sample is compared with that of a known normal sample or a predetermined value obtained from known normal samples. In yet another aspect of the invention, a sequence that is not likely to be methylated is also amplified and detected for comparison with the amplified methylated sequence.

In another aspect of the invention, methylation status is determined via quantitative real time PCR.

In yet another aspect of the invention, a method for characterizing prostate condition includes the step of first testing the patient with a screening assay such as a standard PSA assay. Those patients with concentrations of the markers that are not indicative of a condition that is likely to be cancerous but which is above a normal level are tested for methylation of a prostate cancer marker such as GSTP1, APC, RASSF1A, 15-LO-1, or CDH1. Those patients showing a methylation level beyond a predetermined level are biopsied. In a preferred aspect of this method, the methylation assay is conducted on patients having a PSA level greater than or equal to 2.5 ng/ml. Alternatively, methylation assays are conducted on those with PSA levels of 2-4.

In yet another aspect, the invention is a kit useful for the detection of a methylated nucleic acid. The kit includes one or more containers; a first container containing a reagent that modifies unmethylated cytosine and a second container containing a reagent that primes amplification of CpG-containing nucleic acid, wherein the reagent distinguishes between modified methylated and nonmethylated nucleic acid. The kit contains instructions to conduct the assay on patients with prostate samples assessed as having a Gleason score of 7 or higher. In another embodiment the instructions provide that the assay is run on patients with samples assessed as having a Gleason score greater than 7.

### DETAILED DESCRIPTION OF THE INVENTION

Gleason scores are determined on prostate tissue samples obtained from resection or biopsy. Two samples of abnormal tissue patterns are usually analyzed and their individual score is added together. Methods for sampling and assigning Gleason scores are now well known and widely practiced.

In some methods of the invention, a Gleason score is determined for a prostate cancer patient, a patient being treated for prostate cancer, or a person suspected of having prostate cancer. If the Gleason score is 7 or higher, the patient is tested to determine the methylation status of a nucleic acid that corresponds to a gene whose methylation status correlates with prostate cancer aggressiveness or progression. In the kits of the invention, instructions are provided so that methylation status of a patient is determined for patients for whom a Gleason score of 7 or higher is adduced. In other kits of the invention, instructions are provided so that methylation status of a patient is determined for patients for whom a Gleason score greater than is adduced.

A nucleic acid corresponds to a gene whose methylation status correlates with prostate cancer when methylation status of such a gene provides information about prostate cancer and the sequence is a coding portion of the gene or its complement, a representative portion of the gene or its complement, a promoter or regulatory sequence for the gene or its complement, a sequence that indicates the presence of the gene or its complement, or the full length sequence of the gene or its complement. Such nucleic acids are referred to as Markers in this specification. Markers correspond to the following genes: GSTP1 (Seq. ID. No. 59), RASSF1A (Seq. ID. No. 69), APC (Promoter= Seq. ID. No. 64, Gene= Seq. ID. No. 65), 15-LO-1 (Seq. ID. No. 56), and CDH1 (Seq. ID. No.57). Other sequences of interest include constitutive genes useful as assay controls such as beta-Actin (Seq. ID. No.60 and 61) and PTGS2 (Promoter= Seq. ID. No.66, Gene= Seq. ID. No. 67).

Assays for detecting hypermethylation include such techniques as MSP and restriction endonuclease analysis. The promoter region is a particularly noteworthy target for detecting such hypermethylation analysis. Sequence analysis of the promoter region of GSTP1 shows that nearly 72% of the nucleotides are CG and about 10% are CpG dinucleotides.

The invention includes determining the methylation status of certain regions of the Markers in a tissue or other biological sample of a subject in which the DNA associated with prostate cancer is amplified and detected. Since a decreased level of the protein encoded by the Marker (i.e., less transcription) is often the result of hypermethylation of a particular region such as the promoter, it is desirable to determine whether such regions are hypermethylated. This is seen most demonstrably in the case of the GSTP1 gene. Hypermethylated regions are those that are methylated to a statistically significant greater degree in samples from diseased tissue as compared to normal tissue.

For purposes of the invention, a nucleic acid probe or reporter specific for certain Marker regions is used to detect the presence of methylated regions of the Marker gene in biological fluids or tissues including prostate tissue, urine, urethral washings, blood, blood components such as serum, ejaculate, and other samples from which prostate proteins could be expected. Oligonucleotide primers based on certain portions of the Marker sequence are particularly useful for amplifying DNA by techniques such as PCR. Any specimen containing a detectable amount of the relevant polynucleotide can be used. Urine and prostate tissue are the preferred samples for determining methylation status. Preferably the sample contains epithelial cells.

Some of the primers/probes or reporter reagents of the invention are used to detect methylation of expression control sequences of the Marker genes. These are nucleic acid sequences that regulate the transcription and, in some cases, translation of the nucleic acid sequence. Thus, expression control sequences can include sequences involved with promoters, enhancers, transcription terminators, start codons (i.e., ATG), splicing signals for introns, maintenance of the correct reading frame of that gene to permit proper translation of the mRNA, and stop codons.

The GSTP 1 promoter is an expression control sequence exemplary of a useful Marker. It is a polynucleotide sequence that can direct transcription of the gene to produce a glutathione-s-transferase protein. The promoter region is located upstream, or 5' to the structural gene. It may include elements which are sufficient to render promoter-dependent gene expression controllable for cell-type specific, tissue-specific, or inducible by external signals or agents; such elements may be located in the 5' or 3' regions of the of the polynucleotide sequence.

One method of the invention includes contacting a target cell containing a Marker with a reagent that binds to the nucleic acid. The target cell component is a nucleic acid such as DNA or RNA. The reagents can include probes and primers such as PCR or MSP primers or other molecules configured to amplify and detect the target sequence. For example, the reagents can include priming sequences combined with or bonded to their own reporter segments such as those referred to as Scorpion reagents or Scorpion reporters and described in US Patents 6,326,145 and 6,270,967 to Whitcombe *et. al.* (incorporated herein by reference in their entirety). Though they are not the same, the terms "primers" and "priming sequences" may be used in this specification to refer to molecules or portions of molecules that prime the amplification of nucleic acid sequences.

One sensitive method of detecting methylation patterns involves combining the use of methylation-sensitive enzymes and the polymerase chain reaction (PCR). After digestion of DNA with the enzyme, PCR will amplify from primers flanking the restriction site only if DNA cleavage was prevented by methylation. Exemplary target regions to which PCR primers of the invention are designed include primers which flank the region that lies approximately between -71 and+59 bp according to genomic positioning number of M24485 (Genbank) from the transcription start site of GSTP1.

The method of the invention can also include contacting a nucleic acid-containing specimen with an agent that modifies unmethylated cytosine; amplifying the CpG-containing nucleic acid in the specimen by means of CpG-specific oligonucleotide primers; and detecting the methylated nucleic acid. The preferred modification is the conversion of unmethylated cytosines to another nucleotide that will distinguish the unmethylated from the methylated cytosine. Preferably, the agent modifies unmethylated cytosine to uracil and is sodium bisulfite, however, other agents that modify unmethylated cytosine, but not methylated cytosine can also be used. Sodium bisulfite (NaHSO₃) modification is most preferred and reacts readily with the 5,6-double bond of cytosine, but poorly with methylated cytosine. Cytosine reacts with the bisulfite ion to form a sulfonated cytosine reaction intermediate susceptible to deamination, giving rise to a sulfonated uracil. The sulfonate group can be removed under alkaline conditions, resulting in the formation of uracil. Uracil is recognized as a thymine by Taq polymerase and therefore upon PCR, the resultant product contains cytosine only at the position where 5-methylcytosine occurs in the starting template. Scorpion reporters and reagents and other detection systems similarly distinguish modified from unmodified species treated in this manner.

The primers used in the invention for amplification of a CpG-containing nucleic acid in the specimen, after modification (e.g., with bisulfite), specifically distinguish between untreated DNA, methylated, and non-methylated DNA. In methylation specific PCR (MSPCR), primers or priming sequences for the non-methylated DNA preferably have a T in the 3' CG pair to distinguish it from the C retained in methylated DNA, and the compliment is designed for the antisense primer. MSP primers or priming sequences for non-methylated DNA usually contain relatively few Cs or Gs in the sequence since the Cs will be absent in the sense primer and the Gs absent in the antisense primer (C becomes modified to U (uracil) which is amplified as T (thymidine) in the amplification product).

The primers of the invention are oligonucleotides of sufficient length and appropriate sequence so as to provide specific initiation of polymerization on a significant number of nucleic acids in the polymorphic locus. When exposed to appropriate probes or reporters, the sequences that are amplified reveal methylation status and thus diagnostic information.
Preferred primers are most preferably eight or more deoxyribonucleotides or ribonucleotides capable of initiating synthesis of a primer extension product, which is substantially complementary to a polymorphic locus strand. Environmental conditions conducive to synthesis include the presence of nucleoside triphosphates and an agent for polymerization, such as DNA polymerase, and a suitable temperature and pH. The priming segment of the primer or priming sequence is preferably single stranded for maximum efficiency in amplification, but may be double stranded. If double stranded, the primer is first treated to separate its strands before being used to prepare extension products. The primer must be sufficiently long to prime the synthesis of extension products in the presence of the inducing agent for polymerization. The exact length of primer will depend on factors such as temperature, buffer, and nucleotide composition. The oligonucleotide primers most preferably contain about 12-20 nucleotides although they may contain more or fewer nucleotides, preferably according to well known design guidelines or rules.

Primers are designed to be substantially complementary to each strand of the genomic locus to be amplified and include the appropriate G or C nucleotides as discussed above. This means that the primers must be sufficiently complementary to hybridize with their respective strands under conditions that allow the agent for polymerization to perform. In other words, the primers should have sufficient complementarity with the 5' and 3' flanking sequence(s) to hybridize and permit amplification of the genomic locus.

The primers are employed in the amplification process. That is, reactions (preferably, an enzymatic chain reaction) that produce greater quantities of target locus relative to the number of reaction steps involved. In a most preferred embodiment, the reaction produces exponentially greater quantities of the target locus. Reactions such as these include the PCR reaction. Typically, one primer is complementary to the negative (-) strand of the locus and the other is complementary to the positive (+) strand. Annealing the primers to denatured nucleic acid followed by extension with an enzyme, such as the large fragment of DNA Polymerase I (Klenow) and nucleotides, results in newly synthesized + and - strands containing the target locus sequence. The product of the chain reaction is a discrete nucleic acid duplex with termini corresponding to the ends of the specific primers employed.

The primers may be prepared using any suitable method, such as conventional phosphotriester and phosphodiester methods including automated methods. In one such automated embodiment, diethylphosphoramidites are used as starting materials and may be synthesized as described by Beaucage, et at. (Tetrahedron Letters, 22:1859-1862, 1981). A method for synthesizing oligonucleotides on a modified solid support is described in U.S. Pat. No. 4,458,066.

Any nucleic acid specimen, in purified or non-purified form, can be utilized as the starting nucleic acid or acids, provided it contains, or is suspected of containing, the specific nucleic acid sequence containing the target locus (e.g., CpG). Thus, the process may employ, for example, DNA or RNA, including messenger RNA. The DNA or RNA may be single stranded or double stranded. In the event that RNA is to be used as a template, enzymes, and/or conditions optimal for reverse transcribing the template to DNA would be utilized. In addition, a DNA-RNA hybrid containing one strand of each may be utilized. A mixture of nucleic acids may also be employed, or the nucleic acids produced in a previous amplification reaction herein, using the same or different primers may be so utilized. The specific nucleic acid sequence to be amplified, i.e., the target locus, may be a fraction of a larger molecule or can be present initially as a discrete molecule so that the specific sequence constitutes the entire nucleic acid.

The nucleic acid-containing specimen used for detection of methylated CpG may be tissue (particularly, prostate tissue and lymphatic tissue), blood or blood components, lymph, urine, urethral washings, ejaculate or other biological samples and may be extracted by a variety of techniques such as that described by Maniatis, et al. (Molecular Cloning: A Laboratory Manual, Cold Spring Harbor, N.Y., pp 280, 281, 1982).

If the extracted sample is impure, it may be treated before amplification with an amount of a reagent effective to open the cells, fluids, tissues, or animal cell membranes of the sample, and to expose and/or separate the strand(s) of the nucleic acid(s). This lysing and nucleic acid denaturing step to expose and separate the strands will allow amplification to occur much more readily.

Where the target nucleic acid sequence of the sample contains two strands, it is necessary to separate the strands of the nucleic acid before it can be used as the template. Strand separation can be effected either as a separate step or simultaneously with the synthesis of the primer extension products. This strand separation can be accomplished using various suitable denaturing conditions, including physical, chemical or enzymatic means. One physical method of separating nucleic acid strands involves heating the nucleic acid until it is denatured. Typical heat denaturation may involve temperatures ranging from about 80 to 105°C for up to 10 minutes. Strand separation may also be induced by an enzyme from the class of enzymes known as helicases or by the enzyme RecA, which has helicase activity, and in the presence of riboATP, is known to denature DNA. Reaction conditions that are suitable for strand separation of nucleic acids using helicases are described by Kuhn Hoffmann-Berling (CSH-Quantitative Biology, 43:63, 1978). Techniques for using RecA are reviewed in C. Radding (Ann. Rev. Genetics, 16:405-437, 1982). Refinements of these techniques are now also well known.

When complementary strands of nucleic acid or acids are separated, regardless of whether the nucleic acid was originally double or single stranded, the separated strands are ready to be used as a template for the synthesis of additional nucleic acid strands. This synthesis is performed under conditions allowing hybridization of primers to templates to occur. Generally synthesis occurs in a buffered aqueous solution, preferably at a pH of 7-9, most preferably about 8. A molar excess (for genomic nucleic acid, usually about 10⁸:1, primer:template) of the two oligonucleotide primers is preferably added to the buffer containing the separated template strands. The amount of complementary strand may not be known if the process of the invention is used for diagnostic applications, so the amount of primer relative to the amount of complementary strand cannot always be determined with certainty. As a practical matter, however, the amount of primer added will generally be in molar excess over the amount of complementary strand (template) when the sequence to be amplified is contained in a mixture of complicated long-chain nucleic acid strands. A large molar excess is preferred to improve the efficiency of the process.

The deoxyribonucleoside triphosphates dATP, dCTP, dGTP, and dTTP are added to the synthesis mixture, either separately or together with the primers, in adequate amounts and the resulting solution is heated to about 90-100°C for up to 10 minutes, preferably from 1 to 4 minutes. After this heating period, the solution is allowed to cool to room temperature, which is preferable for the primer hybridization. To the cooled mixture is added an appropriate agent for effecting the primer extension reaction (the "agent for polymerization"), and the reaction is allowed to occur under conditions known in the art. The agent for polymerization may also be added together with the other reagents if it is heat stable. This synthesis (or amplification) reaction may occur at room temperature up to a temperature at which the agent for polymerization no longer functions.

The agent for polymerization may be any compound or system that will function to accomplish the synthesis of primer extension products, preferably enzymes. Suitable enzymes for this purpose include, for example, E. coli DNA polymerase 1, Klenow fragment of E. coli DNA polymerase I, T4 DNA polymerase, other available DNA polymerases, polymerase mutants, reverse transcriptase, and other enzymes, including heat-stable enzymes (e.g., those enzymes which perform primer extension after being subjected to temperatures sufficiently elevated to cause denaturating). A preferred agent is Taq polymerase. Suitable enzymes will facilitate combination of the nucleotides in the proper manner to form the primer extension products complementary to each locus nucleic acid strand. Generally, the synthesis will be initiated at the 3' end of each primer and proceed in the 5' direction along the template strand, until synthesis terminates, producing molecules of different lengths. There may be agents for polymerization, however, which initiate synthesis at the 5' end and proceed in the other direction, using the same process as described above.

Most preferably, the method of amplifying is by PCR. Alternative methods of amplification can also be employed as long as the methylated and non-methylated loci amplified by PCR using the primers of the invention is similarly amplified by the alternative means.

The amplified products are preferably identified as methylated or non-methylated with a probe or reporter specific to the product as described in US Patent 4,683,195 to Mullis et. al.*,* incorporated herein by reference in its entirety. Advances in the field of probes and reporters for detecting polynucleotides are well known to those skilled in the art. Optionally, the methylation pattern of the nucleic acid can be confirmed by other techniques such as restriction enzyme digestion and Southern blot analysis. Examples of methylation sensitive restriction endonucleases which can be used to detect 5'CpG methylation include SmaI, SacII, EagI, MspI, HpaII, BstUI and BssHII.

In another aspect of the invention a methylation ratio is used. This can be done by establishing a ratio between the amount of amplified methylated species of Marker attained and the amount of amplified reference Marker or non-methylated Marker region amplified. This is best done using quantitative real-time PCR. Ratios above an established or predetermined cutoff or threshold are considered hypermethylated and indicative of having a proliferative disorder such as cancer (prostate cancer in the case of GSTP1). Cutoffs are established according to known methods in which such methods are used for at least two sets of samples: those with known diseased conditions and those with known normal conditions. The reference Markers of the invention can also be used as internal controls. The reference Marker is preferably a gene that is constitutively expressed in the cells of the samples such as Beta Actin.

Established or predetermined values (cutoff or threshold values) are also established and used in methods according to the invention in which a ratio is not used. In this case, the cutoff value is established with respect to the amount or degree of methylation relative to some baseline value such as the amount or degree of methylation in normal samples or in samples in which the cancer is clinically insiginificant (is known not to progress to clinically relevant states or is not aggressive). These cutoffs are established according to well-known methods as in the case of their use in methods based on a methylation ratio.

The inventive methods and kits can include steps and reagents for multiplexing. That is, more than one Marker can be assayed at a time.

Since a decreased level of transcription of the gene associated with the Marker is often the result of hypermethylation of the polynucleotide sequence and/or particular elements of the expression control sequences (e.g., the promoter sequence), primers prepared to match those sequences were prepared. Accordingly, the invention provides methods of detecting or diagnosing a cell proliferative disorder by detecting methylation of particular areas within the expression control or promoter region of the Markers. Probes useful for detecting methylation of these areas are useful in such diagnostic or prognostic methods. Preferred molecules for the detection of Markers are shown below. The short name for the Marker gene is shown in parentheses along with the type of detection system employed. Antisense only refers to the orientation of the primer that is so designated in relationship to the priming sequence of the other member of the pair with which it is associated. It is not necessarily antisense with respect to genomic DNA.
**SEQ ID NO. 1 (GSTP1 SCORPION):**
   CCCCGAACGTCGACCGCTCGGGG-BHQ-HEG-CGATTTCGGGGATTTTAGGGCGT
**SEQ ID NO. 2 (GSTP1 SCORPION Antisense Primer):**
   AAAATCCCGCGAACTCCCGCC
**SEQ ID NO. 3 (GSTP1 SCORPION):**
   CCCGAACGTCGACCGCTTTCGGG-BHQ-HEG-CGATTTCGGGGATTTTAGGGCGT
**SEQ ID NO. 4 (GSTP1 SCORPION Antisense Primer):**
   AAAATCCCGCGAACTCCCGCC
**SEQ ID NO. 5 (GSTP1 SCORPION):**
   CGGCGGGAGTTCGCGGGCGCCG-BHQ-HEG-ACTAAATCACGACGCCGACCGC
**SEQ ID NO. 6 (GSTP1 SCORPION Antisense Primer):**
   CGGTTAGTTGCGCGGCGATTTC
**SEQ ID NO. 7 (GSTP1 SCORPION):**
   CGGGAGTTCGCGGGTCCCG-BHQ-HEG-ACTAAATCACGACGCCGACCGC
**SEQ ID NO. 8 (GSTP1 SCORPION Antisense Primer):**
   CGGTTAGTTGCGCGGCGATTTC
**SEQ ID NO. 9 (GSTP1 SCORPION):**
   GTGGTTGATGTTTGGGGTATCAACCAC-BHQ-HEG-AATCCCACAAACTCCCACCAACC
**SEQ ID NO. 10 (GSTP1 SCORPION Antisense Primer):**
   GTGGTGATTTTGGGGATTTTAGGGTGT
**SEQ ID NO. 11 (GSTP1 SCORPION):**
   ACCCCAGTGGTTGATGTTTGGGGT-BHQ-HEG-AATCCCACAAACTCCCACCAACC
**SEQ ID NO. 12 (GSTP1 SCORPION Antisense Primer):**
   GTGGTGATTTTGGGGATTTTAGGGTGT 
SEQ ID NO. 13 (GSTP1 SCORPION):
CCCCACAGGTTGGTGGGAGTTTGTGGGG-BHQ-HEG-
CCCAATACTAAATCACAACACCAACCAC
**SEQ ID NO. 14 (GSTP1 SCORPION Antisense Primer):**
   TGGTTAGTTGTGTGGTGATTTTGGGGA
**SEQ ID NO. 15 (GSTP1 SCORPION):**
   CCCCGAACGTCGACCGCTCGGGG-BHQ-HEG- CGATTTCGGGGATTTTAGGGCGT
**SEQ ID NO. 16 (GSTP1 SCORPION Antisense Primer):**
   AAAATCCCGCGAACTCCCGCC 
SEQ ID NO. 17 (GSTP1 SCORPION):
CGCACGCCGAACGTCGACCGCAAACGTGCG-BHQ-HEG-
CGATTTCGGGGATTTTAGGGCGT
**SEQ ID NO. 18 (GSTP1 SCORPION Antisense Primer):**
   AAAATCCCGCGAACTCCCGCC
**SEQ ID NO. 19 (GSTP1 SCORPION):**
   CGCACGGCGAACTCCCGCCGACGTGCG BHQ-HEG-TGTAGCGGTCGTCGGGGTTG
**SEQ ID NO. 20 (GSTP1 SCORPION Antisense Primer):**
   GCCCCAATACTAAATCACGACG 
SEQ ID NO. 21 (GSTP1 SCORPION):
CCGACGCACAAAAAAACACCCTAAAATCCGTCGG-BHQ-HEG-
GGTTAGTTGTGTGGTGATTTT
**SEQ ID NO. 22 (GSTP1 SCORPION Antisense Primer):**
   CACAACACCAACCACTCTTC
**SEQ ID NO. 23 (GSTP1 TAQMAN PRIMER):**
   CGTGATTTAGTATTGGGGCGGAGCGGGGC
**SEQ ID NO. 24 (GSTP1 TAQMAN PRIMER):**
   ATCCCCGAAAAACGAACCGCGCGTA
**SEQ ID NO. 25 (GSTP1 TAQMAN PROBE):**
   TCGGAGGTCGCGAGGTTITCGTTGGA
**SEQ ID NO. 26 (GSTP1 SCORPION):**
   CGGCCCTAAAACCGCTACGAGGGCCG-BHQ-HEG-GAAGCGGGTGTGTAAGTTTCGG
**SEQ ID NO. 27 (GSTP1 SCORPION Antisense Primer):**
   ACGAAATATACGCAACGAACTAACGC
**SEQ ID NO. 28 (GSTP1 SCORPION):**
   CCGGTCGCGAGGTTTTCGACCGG-BHQ-HEG-CCGAAAAACGAACCGCGCGTA
**SEQ ID NO. 29 (GSTP1 SCORPION Antisense Primer):**
   GGGCGGGATTATTTTTATAAGGTTCGG
**SEQ ID NO. 30 (RASSF1A SCORPION):**
   GCCGCGGTTTCGTTCGGTTCGCGGC-BHQ-HEG-CCCGTACTTCGCTAACTTTAAACG
**SEQ ID NO. 31 (RASSF1A SCORPION Antisense Primer):**
   GCGTTGAAGTCGGGGTTC
**SEQ ID NO. 32 (RARB2 SCORPION):**
   CGGCGCCCGACGATACCCAAAGCGCCG-BHQ-HEG-AACGCGAGCGATTCGAGTAG
**SEQ ID NO. 33 (RARB2 SCORPION Antisense Primer):**
   CTTACAAAAAACCTTCCGAATACG
**SEQ ID NO. 34 (APC SCORPION):**
   GCCGGCGGGTTTTCGACGGGCCGGC-BHQ-HEG-CGAACCAAAACGCTCCCCA
**SEQ ID NO. 35 (APC SCORPION Antisense Primer):**
   GTCGGTTACGTGCGTTTATATTTAG
**SEQ ID NO. 36 (ACTIN SCORPION):**
   GCGCCCAACCGCACAAGGGCGC-BHQ-HEG-GGGTATATTTTCGAGGGGTACG
**SEQ ID NO. 37 (ACTIN SCORPION Antisense Primer):**
   CGACCCGCACTCCGCAAT
**SEQ ID NO. 38(ACTIN SCORPION):**
   CCGCGCATCACCACCCCACACGCGCGG-BHQ-HEG-GGAGTATATAGGTTGGGGAAGTTTG
**SEQ ID NO. 39 (ACTIN SCORPION Antisense Primer):**
   AACACACAATAACAAACACAAATTCAC
**SEQ ID NO. 40 (ACTIN SCORPION):**
   CCCGGCTAAACCCACCATCCAGCCGGG-BHQ-HEG-GGGAGGGTAGTTTAGTTGTGGTT
**SEQ ID NO. 41 (ACTIN SCORPION Antisense Primer):**
   CAAAACAAAAAAACTAAATCTACACAACC
**SEQ 1D NO. 42 (ACTIN SCORPION):**
   CCGCGGAACATTCAACTCAACCGCGG-BHQ-HEG-GGAGGAGGAAGGTAGGTTTTT
**SEQ ID NO. 43 (ACTIN SCORPION Antisense Primer):**
   ACATACAACAATCAATAACATAAAACCAC
**SEQ ID NO. 44 (PTGS2/COX2 SCORPION):**
   CACGCCGCCGTATCTAGGCGTG-BHQ-HEG-GTTTGTTTCGACGTGATTITITCGA
**SEQ ID NO. 45 (PTGS2/COX2 Antisense Primer):**
   GCAAAAAATCCCCTCTCCCGC
**SEQ ID NO. 46 (PTGS2/COX2 SCORPION):**
   GCCGCGCACAAATTTCCGCGGC-BHQ-HEG-GAATTGGTTTTCGGAAGCGTTCG
**SEQ ID NO. 47 (PTGS2/COX2 Antisense Primer):**
   CCCGAATTCCACCGCC
**SEQ ID NO. 48 (PTGS2/COX2 SCORPION):**
   GGCGGAACGCACAAATTTCCGCC-BHQ-HEG-GAATTGGTTTTCGGAAGCGTTCG
**SEQ ID NO. 49 (PTGS2/COX2 Antisense Primer):**
   CCCGAATTCCACCGCC
**SEQ ID NO. 50 (PTGS2/COX2 SCORPION):**
   TGCCGCCGCCGTATCTAATGGCGGCA-BHQ-HEG-GTTTGTTTCGACGTGATTTTTTCGA
**SEQ ID NO. 51 (PTGS2/COX2 Antisense Primer):**
   GCAAAAAATCCCCTCTCCCGC
**SEQ ID NO. 52 (CDH1 SCORPION):**
   CGCCGAATACGATCGGCG-BHQ-HEG-GTTCGTTTTAGTTCGGTTCGA
**SEQ ID NO. 53 (CDH1 SCORPION Antisense Primer):**
   ACCGAAAACGCCGAACGA
**SEQ ID NO. 54 (15LO1 SCORPION):**
   GGCGGCGTTCGGGCCGCC-HEG-BHQ-CCGTACGAACCACAATCGC
**SEQ ID NO. 55 (15LO1 SCORPION Antisense Primer):**
   GGGGTTTCGTTTTATGTCGGT
BHQ=Black Hole Quencher (BioSearch Technologies, San Fransisco, CA)
HEG= Hexaethylene glycol

The kits of the invention can be configured with a variety of components provided that they all contain at least one primer or probe or a detection molecule (e.g., Scorpion reporter). In one embodiment, the kit includes reagents for amplifying and detecting hypermethylated Marker segments. Optionally, the kit includes sample preparation reagents and /or articles (e.g., tubes) to extract nucleic acids from samples.

In a preferred kit, reagents necessary for one-tube MSP are included such as, a corresponding PCR primer set, a thermostable DNA polymerase, such as Taq polymerase, and a suitable detection reagent(s) such as hydrolysis probe or molecular beacon. In optionally preferred kits, detection reagents are Scorpion reporters or reagents. A single dye primer or a fluorescent dye specific to double-stranded DNA such as ethidium bromide can also be used. The primers are preferably in quantities that yield high concentrations. Additional materials in the kit may include: suitable reaction tubes or vials, a barrier composition, typically a wax bead, optionally including magnesium; necessary buffers and reagents such as dNTPs; control nucleic acid (s) and/or any additional buffers, compounds, co-factors, ionic constituents, proteins and enzymes, polymers, and the like that may be used in MSP reactions. Optionally, the kits include nucleic acid extraction reagents and materials.

In a most preferred kit of the invention, instructions to conduct the assay on patients with prostate samples assessed as having a Gleason score of 7 or higher are provided.

In another kit according to the invention, the instructions are to conduct the assay on patients with samples assessed as having a Gleason score greater than 7. In another kit according the invention, instructions are provided to conduct the assay on patients with a PSA level greater than 2.5 ng/ml and in another kit the instructions are provided to conduct the assay on patients with PSA levels of 2-4 ng/ml. The instructions may also indicate that a positive methylation result should be followed up with a biopsy.

### EXAMPLES

### Example 1: Methylation Testing and Gleason Score

Prostate samples were obtained from patients with known clinical outcomes. Gleason scores were assigned to the samples according to well-known methods. From these samples, 52 were found to have Gleason scores less than 7, 36 had Gleason scores of 7, and 12 had Gleason scores greater than 7.

Methylation assays were conducted on each set using GSTP1 (Seq ID No 19, 20) and APC reagents (Seq ID No 34, 35).

The methylation assays were conducted as follows. Genomic DNA was modified using a commercially available sodium bisulfite conversion reagent kit (Zymo Research, Orange, CA, USA). This treatment converted all Cytosines in unmethylated DNA into Uracil, whereas in methylated DNA only cytosines not preceding guanine were converted into Uracil. All cytosines preceeding guanine (in a CpG dinucletide) remained as cytosine.

Sodium bisulfite modified genomic DNA (100 - 150 ng) was amplified in a 25 µl reaction containing the following components: 67mM Tris pH 8.8,16.6mM (NH₄)₂SO₄, 6.7mM MgCl₂, 10mM beta mercaptoethanol; 1.25mM each dATP, dCTP, dGTP, dTTP, 1 U Hot start Taq DNA Ploymerase, 250 nM Scorpion probe, 250 nM reverse or forward primer (depending on scorpion design), 625 nM of passive reference dye.

The samples were then tested in a quantitative real-time PCR assay on the Cepheid SmartCycler^{®} PCR instrument. The PCR conditions used were:
95°C for 60 sec; then 40 cycles of 95°C for 30sec, 59°C for 30 sec, and a final extension at 72°C for 5 min. Optical data was collected at 59°C for every cycle.

A methylation ratio [(copy # of Marker/copy# of B-actin)X1000] cutoff of 1 was established for GSTP1 and a methylation ratio cutoff of 10 was established for APC. The cutoffs were based on clinically relevant sensitivity and specificity requirements. Results were as shown in the following tables:

**Table 1. GSTP1**

| **Gleason Score** | **No. Samples** | **Methylated** | **Not-Methylated** | **Undetermined** | **Sensitivity** |
|---|---|---|---|---|---|
| <7 | 52 | 30 | 16 | 6 | 57.6 |
| 7 | 36 | 24 | 8 | 4 | 66.6 |
| >7 | 12 | 11 | 1 | 0 | 91.6 |

**Table 2. APC**

| **Gleason Score** | **No. Samples** | **Methylated** | **Not-Methylated** | **Undetermined** | **Sensitivity** |
|---|---|---|---|---|---|
| <7 | 52 | 33 | 12 | 7 | 63.46 |
| 7 | 36 | 25 | 8 | 3 | 69.44 |
| >7 | 12 | 11 | 1 | 0 | 91.6 |

These results show that the methylation assay provides accurate information about the prostate cancer status of patients with Gleason scores above 7. Useful and relatively accurate information is also provided in patients with Gleason scores of 7, particularly when combined with other diagnostic or prognostic information.

There is currently a large dichotomy in the Gleason 6 and 7 populations. Approximately half of these patients have a poor prognosis and half have a good prognosis. Until now, there has been no way to determine who will benefit from more aggressive treatment and who will not. The higher sensitivity of methylation assays in cancers with a Gleason score >7, typically the more aggressive cancers, enables one to predict that a patient with a methylation assay result above the cutoff will have a poor prognosis as a result of an aggressive cancer. The methylation data above would predict that 66-69% of the Gleason 7 patients will have a poor prognosis and should be considered for aggressive treatment while the remaining on-third could go into watchful waiting. Thus, the strong correlation of the positivity in the methylation assay in the Gleason score>7 population (the poor prognosis population) indicates prognostic as well as diagnostic value.

### Example 2: Serum Assay

Serum samples were obtained from patients with known prostate cancer outcomes and from whom biopsy samples were taken and Gleason scores adduced. Among these samples, 55 were from patients with no cancer, 36 were from patients with Gleason scores of 5-6, and 21 were from patients with Gleason scores of 7-8.

Methylation status was determined according to the method of Example 1.

The GSTP 1 Marker correctly detected methylation in 26% the samples from patients with a Gleason score of 7-8 and did not detect methylation in those patients with Gleason scores of 5-6 or who were non-cancerous. The APC Marker correctly detected methylation in 26% of the samples from patients with a Gleason score of 7-8, in up to 9 instances it also detected methylation in patients with a Gleason score of 5-6 or who were non-cancerous. The combined specificity of the two Markers was 84% and sensitivity was 18% with a Gleason score of 5-6 and 38% with a Gleason score of 7-8.

A third and fourth Marker, RASSF1a and RARb2 were then added to the group of Markers used to detect methylation to yield a specificity of 82%, a sensitivity of 25% for Gleason scores of 5-6 and 58% for Gleason scores of 7-8. Thus, the inclusion of additional or different methylation markers can be used to boost sensitivity where serum testing is desired and both sensitivity and specificity requirements are heightened.
Additional Marker testing data is shown and described below.
There were 58 samples including 34 prostate adenocarcinoma (CaP), 24 Prostate Benign (Neg), 6 HG-PIN (Neg), 2 Atrophy (Neg), 4 Atypia (Neg), and 2 Inflamatory (Neg). Three samples were missing a biopsy report and one sample failed test (no Actin-hskg Ct value). Markers for GSTP1, RASSF1, RARB2, APC, CDH1 and 15-LO-1 were used.

Reagents were prepared for the msPCR assays using these Markers are shown in Table 3.

**Table 3**

| **Reagents** | Amount (ul) | Final Conc. |
|---|---|---|
| DNA (ul) | 5.0 | - |
| 10x Roche Buffer (no MgCl) | 2.5 | 1x |
| Faststart Taq 5 U/ul | 0.2 | 0.04U |
| 6.25uM probe -Primer mix | 1 | 0.25uM |
| 25 mM dNTPs | 1.25 | 1.25mM |
| 1mM Rox (1:500dilution) | 1 | 80nM |
| MgCl2 (25mM) | 6.7 | 6.7mM |
| Total reaction | 25.0 | - |

Using 0.15uM final probe-primer concentration for 3 GSTP1 mixtures.
Primer/Probes for the Markers were as follows.
GSTP1: Seq ID No. 26/27; Seq ID No. 28/29; Seq ID No. 19/20
RASSF1: Seq ID No. 30/31
RARB2: Seq ID No. 32/33
APC: Seq ID No. 34/35
CDH1: Seq ID No. 52/53
15-LO-1: Seq ID No. 54/55
Beta Actin: Seq ID No. 38/39

PCR conditions are shown in Table 4:

**Table 4**

| **Parameters** | Time | Cycles |
|---|---|---|
| 95C | 5min | 1 |
| 95C | 30sec | 55 |
| 59C | 30sec | (Optics-on) |
| 72C | 30sec | |
| 72C | 5min | 1 |

Table 5 shows the Ct values with six gene specific markers and one hkg and includes available information of Gleason Score and PAS for 58 samples.

**Table 5**

| Sample | ID | Actin | APC | GSTP1 | Rass | RARb | CDH1 | 15 LO | GS(R/L) | PSA 9ng/ml) |
|---|---|---|---|---|---|---|---|---|---|---|
| Cap | 5 | 27.1 | 35.3 | | | 38.8 | 36.9 | | 7/7 | 10 |
| Cap | 6 | 28.5 | 38.9 | 49.7 | 36.8 | | | | 5/5 | 1 |
| Cap | 8 | 29.8 | | | | | | | 7/6 | 11 |
| Cap | 9 | 28.7 | 37.1 | | | | 48.7 | | 6/0 | 5 |
| Cap | 10 | 29.9 | | 35.8 | | | | 38.5 | 8/9 | 135 |
| Cap | 12 | 24.4 | 37.7 | | | 38.3 | 34.6 | | 0/6-7 | N/A |
| Cap | 13 | 26.4 | 39.4 | | | | 48.1 | | 6/7 | N/A |
| Cap | 15 | 26.6 | 39.6 | | | | 53.9 | | 4/0 | N/A |
| Cap | 16 | 28.7 | 42.7 | 48.5 | | | 40.6 | 40.0 | 0/5 | N/A |
| Cap | 18 | 26.7 | 38.6 | | | | | | 0/7 | N/A |
| Cap | 20 | 26.2 | 38.7 | | | | 40.8 | | N/A | N/A |
| Cap | 22 | 27.4 | 35.3 | | | | | | 6/0 | N/A |
| Cap | 23 | 23.9 | 34.7 | | 37.0 | | | | 0/8 | N/A |
| Cap | 26 | 25.7 | | 40.2 | | | | | 0/6 | N/A |
| Cap | 27 | 26.4 | 36.0 | 31.9 | | | | | N/A | N/A |
| Cap | 32 | 25.1 | 38.8 | 32.6 | | | | | 7/7 | N/A |
| Cap | 1S8LMSB | 28.4 | 34.7 | | | | | | N/A | N/A |
| Cap | AH11BSA | 22.5 | 34.3 | | 38.5 | | | 37.4 | N/A | N/A |
| Cap | SB6JDSC | 23.9 | 41.6 | | | | 41.1 | | N/A | N/A |
| Cap | VGKJASA | 23.2 | | 39.2 | | | | | N/A | N/A |
| Cap | WH24ESB | 25.7 | 36.3 | | | | 42.4 | | N/A | N/A |
| Cap | Y3lG8SC | 22.3 | 36.5 | | | | | | N/A | N/A |
| Cap | 5091 | 26.0 | | | 52.5 | | | | 6 | 0.9 |
| Cap | 5098 | 26.1 | 36.7 | | 49.4 | | | | 6 | 7.4 |
| Cap | 5108 | 23.7 | 37.5 | | | | | | 7 | 2.2 |
| Cap | 5113 | 25.9 | | 40.7 | | | | | 6 | 11.8 |
| Cap | 5115 | 25.9 | 34.8 | | 39.4 | | 43.1 | | 7 | 5.1 |
| Cap | 5129 | 30.4 | | | | | | | 7 | 3.3 |
| Cap | 5133 | 24.2 | 33.6 | | | | | | 6 | 7.4 |
| Cap | 5134 | 26.0 | 36.5 | 51.4 | 52.0 | | | | 6 | 6.2 |
| Cap | 5333 | 27.5 | 40.3 | | 37.4 | | | | 7 | 8.7 |
| Cap | 5343 | 29.5 | | | 48.9 | | 54.6 | | 6 | 7.9 |
| Cap | 5349 | 35.6 | | 41.7 | 49.0 | | | | 6 | 6.8 |
| Cap | 5354 | 28.4 | 34.7 | | | 33.6 | | | 6 | 3.6 |
| HG-PIN | 7 | 27.6 | | 37.9 | | | | | | 9 |
| HG-PIN | 2810 | 29.0 | 38.4 | | 41.0 | | | | | 5.7 |
| HG-PIN | 3002 | 26.9 | 35.8 | 54.9 | | | | | | N/A |
| HG-PIN | 3210 | 25.5 | 37.8 | 39.1 | 44.6 | | 54.8 | | | N/A |
| HG-PIN | 3319 | 23.7 | 37.8 | 47.6 | | | | | | N/A |
| HG-PIN | 4079 | 28.8 | 36.4 | 47.8 | | | | | | 2.2 |
| Benign | 11 | 29.5 | 50.3 | | | | 51.9 | | | N/A |
| Benign | 14 | 31.9 | | | | | 44.1 | | | N/A |
| Benign | 21 | 28.3 | 49.4 | | | | | | | N/A |
| Benign | 3263 | 24.9 | | | | 41.0 | | | | 1.4 |
| Benign | 3602 | 26.0 | 36.2 | 44.3 | 49.0 | | | | | 10.1 |
| Benign | 3836 | 25.3 | 38.3 | | | | | | | N/A |
| Benign | 3882 | 28.3 | 36.2 | 45.9 | | | | | | N/A |
| Benign | 4017 | 27.6 | | | | | | | | 7.3 |
| Benign | 5569 | 28.9 | 28.7 | | 39.3 | | | | | 3.0 |
| Atrophy | 3006 | 27.5 | 39.1 | 47.8 | | 40.9 | | | | N/A |
| Atrophy | 3285 | 26.1 | 38.7 | | | | | | | N/A |
| Atypia | 3358 | 23.5 | | | | 41.9 | 39.1 | | | 2.8 |
| Atypia | 3512 | 26.9 | 37.4 | | | | 48.4 | | | 5.0 |
| Atypia | 3804 | 27.4 | | | | | | 42.4 | | 3.9 |
| Atypia | 4393 | 28.9 | 37.4 | | 48.4 | | | | | 3.8 |
| Inflam | 17 | 29.4 | 38.0 | | 45.7 | | | | | N/A |
| Inflam | 2989 | 29.2 | 38.1 | | 40.0 | | | | | N/A |
| Inflam | 3182 | 25.3 | 37.3 | | | | 45.7 | | | 7.2 |

| | | | | | | | | | | |
|---|---|---|---|---|---|---|---|---|---|---|
| Blank- not determined for Ct after 55 cycles of QMSP. | | | | | | | | | | |

Sensitivity and specificity were determined directly by Ct values shown in Table 6.

**Table 6**

| Ct cutoff setting for 6 or 4 markers | | APC | GSTP1 | Rass | RARb2 | CDH1 | 15 LO |
|---|---|---|---|---|---|---|---|
| Sensitivity | 55% | 37 | 37 | 40 | 40 | 40 | 40 |
| Specificity | 82% | | | | | | |
| Sensitivity | 52% | 37 | 37 | 40 | 40 | not used | not used |
| Specificity | 84% | | | | | | |
| Sensitivity | 39% | 36 | 37 | 39 | 40 | not used | not used |
| Specificity | 95% | | | | | | |
| Sensitivity | 37% | 35 | 37 | 39 | 40 | not used | not used |
| Specificity | 97% | | | | | | |

### Example 3: Urine Assay

Urine samples were obtained from patients with known prostate cancer outcomes and from whom biopsy samples were taken and Gleason scores adduced. Among these samples, 42 were from patients with with Gleason scores of 4-6 and 10 were from patients with Gleason scores of 7-9.

Methylation status was determined according to the method of Example 1 using the Cepheid Smart Cycler PCR instrument.

The combined specificity of the two Markers, GSTP 1 and RARb2 was 89% for post-massage urine samples and 91% for post biopsy samples. Methylation assays with post massage samples were 40% sensitive in those with Gleason scores below 7 and 78% for those with scores greater than 7. Thus, noninvasive sampling can be used in conjunction with the other aspects of the invention.

### Example 4: Serum Assay with PSA Result (Prophetic)

Serum samples are obtained from patients with known prostate cancer outcomes. PSA concentrations are determined according to standard clinical methods. Among these samples, 55 are from patients with no cancer having PSA levels of 1-2 ng/ml, 36 are from patients with PSA levels of 2-4 ng/ml, and 21 are from patients with PSA levels greater than 4. Patients with PSA levels greater than 4 are indicated for biopsies according to well-established clinical guidelines.

The methylation status for patients with PSA levels below 4 are determined according to the method of Example 1.

The GSTP 1 Marker detects methylation in 20% the samples from patients with a PSA level of 2-4. These patients are biopsied and found to have a Gleason score of 7 or greater. Further treatment is likely indicated in these patients. Hypermethylation is not found in any samples from patients with a PSA value less than 2. APC, RASSF1A, 15-LO-1, and CDH1 Markers are used in a separate methylation assays of these patients and 15% of the samples are found to be hypermethylated. These patients are biopsied and found to have a Gleason score of 7 or greater. Further treatment is likely indicated in these patients. The combined specificity of two Markers is 95% and sensitivity is 85% in patients with PSA levels below 4.

A patient with a PSA score that makes the need for biopsy uncertain is stratified according to the outcome of a methylation assay. This can be particularly useful in a watchful-waiting course of therapy or in a therapy monitoring strategy in general. The patient is periodically tested with a non-biopsy assay such as the PSA test and tested for DNA methylation status of prostate Markers when results that would indicate biopsy are ambiguous or difficult to interpret. A methylation result greater than a pre-determined cutoff indicates a biopsy is necessary and that a Gleason score of 7 or greater is likely to be at least one result of such biopsy.

## Claims

1. A method of predicting the recurrence or aggressiveness of prostate cancer comprising, a) determining the Gleason score of a prostate sample, and b) determining the methylation status of a Marker in a biological sample for those patients having a Gleason score of 7 or greater; wherein methylation that exceeds a pre-determined value is indicative of an aggressive or recurrent cancer and methylation that does not exceed such pre-determined value is indicative of indolent cancer.

2. The method according to claim 1 further comprising measuring the presence of a reference Marker.

3. The method according to claim 2 wherein the reference Marker is selected from the group consisting of beta Actin and PTGS2.

4. The method of claim wherein a combination of Markers are used.

5. The method of claim 4 wherein the combination of Markers includes a Marker for GSTP1 and a Marker for APC, RASSF1A, 15-LO-1, or CDH1.

6. The method of claim 1 wherein the sample from which methylation status is determined is prostate tissue.

7. The method of claim 1 wherein the sample from which methylation status is determined is urine, urethral washing, blood, a blood component, ejaculate, or circulating cells.

8. The method of claim 7 wherein said sample is serum or plasma.

9. A kit for conducting an assay to predict the course or aggressiveness of prostate cancer, comprising: nucleic acid amplification and detection reagents and instructions that direct its use in patients in whom a Gleason score of 7 or higher was adduced.

10. The kit of claim 9 wherein the instructions direct the use of the kit in patients in whom a Gleason score greater than 7 was adduced.

11. The kit of claim 9 wherein the reagents include a member of the group consisting of Seq. ID No. 26 and 27.

12. The kit of claim 9 wherein the PCR priming reagents consist essentially of of Seq. ID No. 26 and 27.

13. The kit of claim 9 wherein the reagents include a member of the group consisting of Seq. ID No. 28 and 29.

14. The kit of claim 9 wherein the PCR priming reagents consist essentially of Seq. ID No. 28 and 29.

15. The kit of claim 9 wherein the reagents include a member of the group consisting of Seq. ID No. 32 and 33.

16. The kit of claim 9 wherein the PCR priming reagents consist essentially of Seq. ID No. 32 and 33.

17. The kit of claim 9 wherein the reagents include a member of the group consisting of Seq. ID No. 52 and 53.

18. The kit of claim 9 wherein the PCR priming reagents consist essentially of Seq. ID No. 52 and 53.

19. The kit of claim 9 wherein the reagents include a member of the group consisting of Seq. ID No. 54 and 55.

20. The kit of claim 9 wherein the PCR priming reagents consist essentially of Seq. ID No. 54 and 55

21. The kit of claim 9 wherein the reagents detect the hypermethylation of a gene selected from the group consisting of GSTP1, APC, RASSF1A, 15-LO-1, and CDH1.

22. The kit of claim 9 further comprising reagents for amplifying and detecting the presence of a constitutively expressed gene.

23. The method of claim 1 further comprising establishing a methylation ratio and determining whether the methylation ratio exceeds a cutoff value.

24. The method of claim 1 for use in therapy monitoring.

25. A method of determining whether a patient should undergo prostate biopsy testing comprising: a) determining the level of PSA in a patient sample, and b) determining the methylation status of a Marker in a biological sample for those patients with a PSA level greater than 2 and less than or equal to 4 ng/ml; wherein patients with methylation values that exceeds a pre-determined value are selected for biopsy testing.

26. The method according to claim 25 further comprising measuring the presence of a reference Marker.

27. The method according to claim 26 wherein the reference Marker is selected from the group consisting of beta Actin and PTGS2.

28. The method of claim 25 wherein a combination of Markers are used.

29. The method of claim 28 wherein the combination of Markers includes a Marker for GSTP1 and a Marker for APC, RASSF1A, 15-LO-1, or CDH1.

30. The method of claim 25 wherein the sample from which methylation status is determined is urine, urethral washing, blood, a blood component, ejaculate, or circulating cells.

31. The method of claim 30 wherein said sample is serum or plasma.

32. A kit for conducting an assay to determine whether a patient should undergo prostate biopsy testing comprising: nucleic acid amplification and detection reagents and instructions that direct its use in patients in whom a PSA level between 2 and 4 ng/ml is found.
